# EUROPÄISCHE PATENTSCHRIFT

(11) **EP 1 301 485 B1**
(45) Veröffentlichungstag und Bekanntmachung des Hinweises auf die Patenterteilung: **27.06.2007**
(21) Anmeldenummer: 01969410.8
(22) Anmeldetag: 18.07.2001
(51) Int. Cl.: C07D 219/04, A61K 31/473, A61P 35/00

(54) **ACRIDIN-DERIVATE UND DEREN VERWENDUNG ALS ARZNEIMITTEL**
NOVEL HETEROARYL DERIVATIVES AND THE USE THEREOF AS PHARMACEUTICALS
DERIVES ACRIDINE ET UTILISATION EN TANT QU'AGENTS PHARMACEUTIQUES

(30) Priorität: 21.07.2000 DE 10035927
(43) Veröffentlichungstag der Anmeldung: 16.04.2003
(73) Patentinhaber: Zentaris GmbH, 60314 Frankfurt/Main (DE)
(72) Erfinder: EMIG, Peter, 63486 Bruchköbel (DE); GÜNTHER, Eckhard, 63477 Maintal (DE); BAASNER, Silke, 61137 Schöneck (DE); BACHER, Gerald, 82110 Germering (DE); BECKERS, Thomas, 78464 Konstanz (DE); AUE, Beate, 63762 Großostheim/Ringheim (DE); NICKEL, Bernd, 64367 Mühltal (DE)
(86) Internationale Anmeldenummer: PCT/EP2001/008263
(87) Internationale Veröffentlichungsnummer: WO 2002/008194

(56) Entgegenhaltungen:
- WO-A-98/00402
- EBCID , M. Y. ET AL.: "Synthesis and antitumor activity of some 4-{4-[4-(2-hydroxyethyl)piperazine-1-carbo nyl]acridin-9-ylamino}-benzenesulfonamide derivatives" BULL. FAC. PHARM., Bd. 32, Nr. 3, 1994, Seiten 361-368, XP001037725
- ATWEEL G J ET AL: "POTENTIAL ANTITUMOR AGENTS. 50. IN VIVO SOLID-TUMOR ACTIVITY OF DERIVATIVES OF N-[2-(DIMETHYLAMINO)ETHYL]ACRIDINE-4-CARBO XAMIDE" JOURNAL OF MEDICINAL CHEMISTRY, AMERICAN CHEMICAL SOCIETY. WASHINGTON, US, Bd. 30, Nr. 4, 1987, Seiten 664-669, XP002051603 ISSN: 0022-2623

## Beschreibung

Die Erfindung betrifft neue Heteroaryl-Derivate der allgemeinen Formel 1, deren Herstellung und Verwendung als Arzneimittel, insbesondere zur Behandlung von Tumoren.

Gemäß einem Aspekt der Erfindung werden neue Acridin-Derivate gemäß der allgemeinen Formel 1 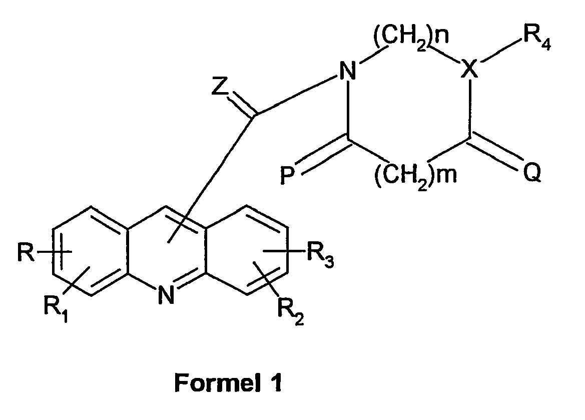 worin
- R, R₁, R₂, R₃: wahlweise an den Acridin-Kohlenstoffatomen C₁ bis C₉ gebunden sein können, gleich oder verschieden sind und unabhängig voneinander Wasserstoff, geradkettiges oder verzweigtes (C₁-C₈)-Alkyl, (C₃-C₇)-Cycloalkyl, geradkettigtes oder verzweigtes (C₁-C₈)-Alkylcarbonyl, vorzugsweise Acetyl, geradkettiges oder verzweigtes (C₁-C₈)-Alkoxy, Halogen, Aryl-(C₁-C₈)-alkoxy, vorzugsweise Benzyloxy oder Phenyl-ethyloxy, Nitro, Amino, Mono-(C₁-C₄)-Alkylamino, Di-(C₁-C₄)-Alkylamino, (C₁-C₈)-Alkoxycarbonyl-amino, (C₁-C₆)-Alkoxycarbonylamino-(C₁-C₈)-alkyl, Cyano, geradkettiges oder verzweigtes Cyano-(C₁-C₆)-alkyl, Carboxy, (C₁-C₈)-Alkoxycarbonyl, mit einem oder mehreren Fluoratomen substituiertes (C₁-C₄)-Alkyl, vorzugsweise die Trifluormethylgruppe, Carboxy-(C₁-C₈)-alkyl oder (C₁-C₈)-Alkoxycarbonyl-(C₁-C₆)-alkyl, (C₂-C₆)-Alkenyl, vorzugsweise Allyl, (C₂-C₆)-Alkinyl, vorzugsweise Ethinyl oder Propargyl, geradkettiges oder verzweigtes Cyano-(C₁-C₆)-alkyl, vorzugsweise Cyanomethyl, Aryl, wobei der Arylrest unsubstituiert oder ein-oder mehrfach gleich oder verschieden mit Halogen, geradkettigem oder verzweigtem (C₁-C₈)-Alkyl, (C₃-C₇)-Cycloalkyl, Carboxy, geradkettigem oder verzweigtem (C₁-C₈)-Alkoxycarbonyl, vorzugsweise tert.-Butoxycarbonyl, mit Trifluormethyl, Hydroxy, geradkettigem oder verzweigtem (C₁-C₈)-Alkoxy, vorzugsweise Methoxy oder Ethoxy, Benzyloxy, Nitro, Amino, Mono-(C₁-C₄)-Alkylamino, Di-(C₁-C₄)-Alkylamino, Cyano, geradkettigem oder verzweigtem Cyano-(C₁-C₆)-alkyl substituiert sein kann, bedeuten,
- Z: Sauerstoff oder Schwefel ist, wobei der am Acridin-Heterocyclus substituierte Rest 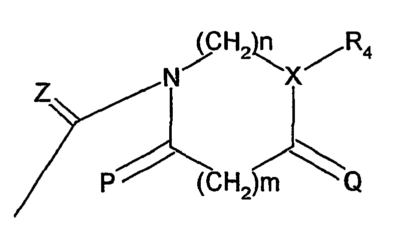 an den C-Atomen C₁-C₉ des Acridin-Ringgerüstes gebunden sein kann;
- P, Q: unabhängig voneinander für Sauerstoff oder jeweils für zwei Wasserstoffatome (also -CH₂-) stehen;
- X: Stickstoff oder C-R₅ ist, wobei R₅ für Wasserstoff oder (C₁-C₆)-Alkyl steht
- n,m: unabhängig voneinander eine ganze Zahl zwischen 0-3 bedeuten, mit der Maßgabe, dass im Falle n=0 X eine CR₅R₆-Gruppe, wobei R₅ und R₆ unabhängig voneinander für Wasserstoff oder (C₁-C₆)-Alkyl stehen, bedeutet und an dem der C=Z-Gruppe benachbarten Stickstoff-Atom ein Wasserstoff-Atom oder eine (C₁-C₆)-Alkylgruppe substituiert ist,
- R₄: einen geradkettigen oder verzweigten (C₁-C₂₀)-Alkyl-Rest, welcher gesättigt oder mit ein bis drei Doppel- und/oder Dreifachbindungen ungesättigt sein kann und welcher unsubstituiert oder wahlweise an dem gleichen oder verschiedenen C-Atomen mit ein, zwei oder mehreren Aryl, Heteroaryl, Halogen, Cyano, (C₁-C₆)-Alkoxycarbonylamino, (C1-C6)-Alkoxy, Amino, Mono-(C₁-C₄) Alkylamino oder Di- (C₁-C₄)-Alkylamino substituiert sein kann; einen (C₆-C₁₄)-Aryl-Rest, (C₆-C₁₄)-Aryl-(C₁-C₄)-alkyl-Rest oder einen ein oder mehrere Heteroatome ausgewählt aus der Gruppe N, O und S enthaltenden (C₂-C₁₀)-Heteroaryl- oder (C₂-C₁₀)-Heteroaryl-(C₁-C₄)-alkyl-Rest, wobei der (C₁ -C₄)-alkyl-Rest unsubstituiert oder ein- mehrfach gleich oder verschieden mit (C₁-C₆)-Alkyl, Halogen oder Oxo (=O) substituiert sein kann und der (C₆-C₁₄)-Aryl- oder (C₂-C₁₀)-Heteroaryl -Rest unsubstituiert oder ein- oder mehrfach gleich oder verschieden mit geradkettigem oder verzweigtem (C₁-C₈)-Alkyl, (C₃-C₇)-Cycloalkyl, Halogen, Cyano, (C₁-C₆)-Alkoxycarbonylamino, (C₁-C₆)-Alkoxy, Carboxy, (C₁-C₈)-Alkoxycarbonyl, mit einem oder mehreren Fluoratomen substituierten geradkettigem oder verzweigtem (C₁-C₆)-Alkyl, vorzugsweise Trifluormethyl, Hydroxy, geradkettigem oder verzweigtem (C₁-C₈)-Alkoxy, vorzugsweise Methoxy oder Ethoxy, wobei benachbarte Sauerstoffatome auch durch (C₁-C₂)-Alkylen-Gruppen, vorzugsweise eine Methylen-Gruppe verknüpft sein können, Benzyloxy, Nitro, Amino, Mono-(C₁-C₄)-Alkylamino, Di-(C₁-C₄)-Alkylamino, Aryl, das seinerseits unsubstituiert oder ein- oder mehrfach gleich oder verschieden mit geradkettigem oder verzweigtem (C₁-C₈)-Alkyl, (C₃-C₇)-Cycloalkyl, Carboxy, geradkettigem oder verzweigtem (C₁-C₈)-Alkoxycarbonyl, mit Trifluormethyl, Hydroxy, geradkettigem oder verzweigtem (C₁-C₈)-Alkoxy, vorzugsweise Methoxy oder Ethoxy, Benzyloxy, Nitro, Amino, Mono-(C₁-C₄) Alkylamino, Di- (C₁-C₄)-Alkylamino, Cyano, geradkettigem oder verzweigtem Cyano-(C₁-C₆)-alkyl substituiert ist, substituiert sein kann;
sowie deren Struktur- und Stereoisomeren, insbesondere Tautomere, Diastereomere und Enantiomere, und deren pharmazeutisch verträglichen Salzen, insbesondere Säureadditionssalze; bereitgestellt.

So lassen sich beispielsweise die erfindungsgemäßen Verbindungen gemäß der allgemeinen Formel (1), welche ein oder mehrere Chiralitätszentren aufweisen und die als Racemate auftreten, nach an sich bekannten Methoden in ihre optischen lsomeren, also Enantiomere oder Diastereomere auftrennen. Die Trennung kann durch Säulentrennung an chiralen Pasen oder durch Umkristallisation aus einem optisch aktiven Lösungsmittel oder unter Verwendung einer optisch aktiven Säure oder Base oder durch Derivatisierung mit einem optisch aktiven Reagenzes, wie beispielsweise einem optisch aktiven Alkohol, und anschließender Abspaltung des Restes erfolgen.

Des weiteren können die erfindungsgemäßen Acridin-Derivate der allgemeinen Formel (1) in ihre Salze mit anorganischen oder organischen Säuren, insbesondere für die pharmazeutische Anwendung in ihre physiologisch verträglichen Salze, überführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Essigsäure, Weinsäure, Äpfelsäure, Malonsäure, Embonsäure, Trifluoressigsäure oder Maleinsäure in Betracht.

Außerdem lassen sich die erfindungsgemäßen Verbindungen gemäß der Formel (1), falls diese eine ausreichend saure Gruppe wie eine Carboxygruppe enthalten, gewünschtenfalls in ihre Salze mit anorganischen oder organischen Basen, insbesondere für die pharmazeutische Anwendung in ihre physiologisch verträglichen Salze, überführt werden. Als Basen kommen hierbei beispielsweise Natriumhydroxid, Kaliumhydroxid, Calciumhydroxid, Lysin, Cyclohexylamin, Ethanolamin, Diethanolamin und Triethanolamin in Betracht.

Gemäß einer bevorzugten Ausführungsform werden Acridin-Derivate gemäß der allgemeinen Formel 1 bereitgestellt, worin R, R1, R2, R3, X, Z, P, Q, n und m die vorstehend genannten Bedeutungen besitzen und
- R₄: einen geradkettigen oder verzweigten (C₁-C₂₀)-Alkyl-Rest, welcher gesättigt oder mit ein bis drei Doppel- und/oder Dreifachbindungen ungesättigt sein kann und welcher unsubstituiert oder wahlweise an dem gleichen oder verschiedenen C-Atomen mit ein, zwei oder mehreren Aryl, Heteroaryl, Halogen, (C1-C6)-Alkoxy, Amino, Mono-(C₁-C₄) Alkylamino oder Di- (C₁-C₄)-Alkylamino substituiert sein kann;
einen Phenyl-Rest oder einen Naphthyl-Rest, die jeweils unsubstituiert oder ein- oder mehrfach gleich oder verschieden mit geradkettigem oder verzweigtem (C₁-C₈)-Alkyl, (C₃-C₇)-Cycloatkyl, Halogen, Cyano, (C₁-C₆)-Alkoxycarbonylamino, (C₁-C₆)-Alkoxy, Carboxy, (C₁-C₈)-Alkoxycarbonyl, mit einem oder mehreren Fluoratomen substituierten geradkettigem oder verzweigtem (C₁-C₆)-Alkyl, vorzugsweise Trifluormethyl, Hydroxy, geradkettigem oder verzweigtem (C₁-C₈)-Alkoxy, vorzugsweise Methoxy oder Ethoxy, wobei benachbarte Sauerstoffatome auch durch (C₁-C₂)-Alkylen-Gruppen, vorzugsweise eine Methylen-Gruppe verknüpft sein können, Benzyloxy, Nitro, Amino, Mono-(C₁-C₄)-Alkylamino, Di-(C₁-C₄)-Alkylamino, Aryl, das seinerseits unsubstituiert oder ein- oder mehrfach gleich oder verschieden mit geradkettigem oder verzweigtem (C₁-C₈)-Alkyl, (C₃-C₇)-Cycloalkyl, Carboxy, geradkettigem oder verzweigtem (C₁-C₈)-Alkoxycarbonyl, mit Trifluormethyl, Hydroxy, geradkettigem oder verzweigtem (C₁-C₈)-Alkoxy, vorzugsweise Methoxy oder Ethoxy, Benzyloxy, Nitro, Amino, Mono-(C₁-C₄) Alkylamino, Di- (C₁-C₄)-Alkylamino, Cyano, geradkettigem oder verzweigtem Cyano-(C₁-C₆)-alkyl substituiert ist, substituiert sein können,
einen 2-, 4-, 5- oder 6-Pyrimidinyl-Rest oder 2-, 4-, 5- oder 6-Pyrimidinyl-(C₁-C₄)-alkyl-Rest, wobei der (C₁-C₄)-alkyl-Rest unsubstituiert oder ein- mehrfach gleich oder verschieden mit (C₁-C₆)-Alkyl, Halogen oder Oxo (=O) substituiert sein kann und der 2-, 4-, 5- oder 6-Pyrimidinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 3-, 4-, 5-, oder 6-Pyridazinyl-Rest oder 3-, 4-, 5-, oder 6-Pyridazinyl-(C₁-C₄)-alkyl-Rest, wobei der (C₁-C₄)-alkyl-Rest unsubstituiert oder ein- mehrfach gleich oder verschieden mit (C₁-C₆)-Alkyl, Halogen oder Oxo (=O) substituiert sein kann und der 3-, 4-, 5-, oder 6-Pyridazinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 3-, 5,- oder 6-Pyrazinyl-Rest oder 2-, 3-, 5,- oder 6-Pyrazinyl-(C₁-C₄)-alkyl-Rest, wobei der (C₁-C₄)-alkyl-Rest unsubstituiert oder ein- mehrfach gleich oder verschieden mit (C₁-C₆)-Alkyl, Halogen oder Oxo (=O) substituiert sein kann und der 2-, 3-, 5,- oder 6-Pyrazinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 3-, 4-, 5-, 6-, 7-, oder 8-Cinnolinyl-Rest oder 3-, 4-, 5-, 6-, 7-, oder 8- Cinnolinyl-(C₁-C₄)-alkyl-Rest, wobei der (C₁-C₄)-alkyl-Rest unsubstituiert oder ein- mehrfach gleich oder verschieden mit (C₁-C₆)-Alkyl, Halogen oder Oxo (=O) substituiert sein kann und der 3-, 4-, 5-, 6-, 7-, oder 8-Cinnotinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₈)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4-, 5-, 6-, 7-, oder 8-Chinazolinyl-Rest oder 2-, 4-, 5-, 6-, 7-, oder 8-Chinazolinyl-(C₁-C₄)-alkyl-Rest, wobei der (C₁-C₄)-alkyl-Rest unsubstituiert oder ein- mehrfach gleich oder verschieden mit Wasserstoff, (C₁-C₆)-Alkyl, Halogen oder Oxo (=O) substituiert sein kann und der oder 2-, 4-, 5-, 6-, 7-, oder 8-Chinazolinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 3-, 5-, 6-, 7-, oder 8-Chinoxalinyl-Rest 2-, 3-, 5-, 6-, 7-, oder 8-Chinoxalinyl-(C₁-C₄)-alkyl-Rest, wobei der (C₁-C₄)-alkyl-Rest unsubstituiert oder ein- mehrfach gleich oder verschieden mit (C₁-C₆)-Alkyl, Halogen oder Oxo (=O) substituiert sein kann und der oder 2-, 3-, 5-, 6-, 7-, oder8-Chinoxalinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆-Alkylamino, Hydroxy, (C₁-C₆-Alkoxy, Benzyloxy, Carboxy, (C₁-C₆-Alkoxycarbonyl, (C₁-C₆-Alkoxycarbonylamino oder ein- oder mehrfach mit Fluor substituiertes (C₁-C₆-Alkyl, vorzugsweise Trifluormethyl, (C₆-C₁₀-Aryl, oder (C₆-C₁₀-Aryl-(C₁-C₆-alkyl substituiert sein kann;
einen 1-, 4-, 5-, 6-, 7-, oder 8-Phthalazinyl-Rest oder 1-, 4-, 5-, 6-, 7-, oder 8-Phthalazinyl-(C₁ -C₄)-alkyl-Rest, wobei der (C₁ -C₄)-alkyl-Rest unsubstituiert oder ein- mehrfach gleich oder verschieden mit (C₁ -C₆)-Alkyl, Halogen oder Oxo (=O) substituiert sein kann und der oder 1-, 4-, 5-, 6-, 7-, oder 8-Phthalazinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 3-, 4-, 5-, 6-, 7- oder 8-Chinolyl-Rest oder 2-, 3-, 4-, 5-, 6-, 7- oder 8-Chinolyl-(C₁-C₄)-alkyl-Rest, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der 2-, 3-, 4-, 5-, 6-, 7- oder 8-Chinolyl-Rest unsubstituiert oder ein- bis sechsfach gleich oder verschieden mit Wasserstoff, (C₁-C₆)-Alkyl, vorzugsweise Methyl, besonders bevorzugt 2-Methyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 3-, 4-, 5-, 6-, 7- oder 8-lsochinolyl- oder 1-, 3-, 4-, 5-, 6-, 7- oder 8-Isochinolyl-(C₁-C₄)-alkyl-Rest, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der 1-, 3-, 4-, 5-, 6-, 7- oder 8-Isochinolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 6-, 8- oder 9-[9*H*]-Purinyl-Rest oder 2-, 6-, 8- oder 9-[9*H*]-Purinyl-(C₁ -C₄)-alkyl-Rest, wobei der (C₁ -C₄)-alkyl-Rest unsubstituiert oder ein- oder mehrfach gleich oder verschieden mit (C₁ -C₆)-Alkyl, Halogen oder Oxo (=O) substituiert sein kann und der 2-, 6-, 8- oder 9-[9*H*]-Purinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 6-, 7- oder 8-[7*H*]-Purinyl-Rest oder 2-, 6-, 7- oder 8-[7*H*]-Purinyl-(C₁ -C₄)-alkyl-Rest, wobei der (C₁ -C₄)-alkyl-Rest unsubstituiert oder ein- oder mehrfach gleich oder verschieden mit (C₁ -C₆)-Alkyl, Halogen oder Oxo (=O) substituiert sein kann und der 2-, 6-, 7- oder 8-[7*H*]-Purinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 2-, 3-, 4-, 5-, 6-, 7-, 8- oder 9-Acridinyl- oder 1-, 2-, 3-, 4-, 5-, 6-, 7-, 8- oder 9-Acridinyl-(C₁ -C₄)-alkyl-Rest, wobei der (C₁ -C₆)-alkyl-Rest unsubstituiert oder ein- oder mehrfach gleich oder verschieden mit (C₁ -C₆)-Alkyl, Halogen oder Oxo (=O) substituiert sein kann und der 1-, 2-, 3-, 4-, 5-, 6-, 7-, 8- oder 9-Acridinyl-Rest unsubstituiert oder ein- bis achtfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 2-, 3-, 4-, 5-, 6-, 7-, 8- oder 9-Phenanthridinyl- oder 1-, 2-, 3-, 4-, 5-, 6-, 7-, 8- oder 9- Phenanthridinyl-(C₁-C₆)-alkyl-Rest, wobei der (C₁-C₆)-alkyl-Rest unsubstituiert oder ein- oder mehrfach gleich oder verschieden mit Wasserstoff, (C₁-C₆)-Alkyl, Halogen oder Oxo (=O) substituiert sein kann und der 1-, 2-, 3-, 4-, 5-, 6-, 7-, 8- oder 9-Phenanthridinyl-Rest unsubstituiert oder ein- bis achtfach gleich oder verschieden mit (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, (C₆-C₁₀)-Aryl-(C₁-C₆)-alkoxy, vorzugsweise Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 3-, 4-, 5,- oder 6-Pyridyl-Rest, wobei der 2-, 3-, 4-, 5,- oder 6-Pyridinyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 3-, 4-, 5,- oder 6-Pyridyl-(C₁ -C₆)-alkyl-Rest, wobei der (C₁ -C₆)-alkyl-Rest unsubstituiert oder ein- oder mehrfach gleich oder verschieden mit (C₁-C₆)-Alkyl, Halogen oder Oxo (=O) substituiert sein kann und der 2-, 3-, 4-, 5,- oder 6-Pyridinyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 3-, 4,- oder 5-Thienyl-Rest oder 2-, 3-, 4,- oder 5-Thienyl-(C₁-C₆)-alkyl-Rest, wobei der (C₁-C₆)-alkyl-Rest unsubstituiert oder ein- oder mehrfach gleich oder verschieden mit (C₁ -C₆)-Alkyl, Halogen oder Oxo (=O) substituiert sein kann und der 2-, 3-, 4,- oder 5-Thienyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4-, oder 5-Thiazolyl-Rest oder 2-, 4-, oder 5-Thiazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2-, 4-, oder 5-Thiazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 3-, 4-, oder 5-Isothiazolyl-Rest oder 3-, 4-, oder 5-Isothiazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 3-, 4-, oder 5-lsothiazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4-, 5-, 6-, oder 7-Benzthiazolyl-Rest oder 2-, 4-, 5-, 6-, oder 7-Benzthiazolyl (C₁ -C₆)-alkyl-Rest, wobei der (C₁ -C₆)-alkyl-Rest unsubstituiert oder ein- oder mehrfach gleich oder verschieden mit (C₁ -C₆)-Alkyl, Halogen oder Oxo (=0) substituiert sein kann und der 2-, 4-, 5-, 6-, oder 7-Benzthiazolyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 2-, 4-, oder 5-Imidazolyl-Rest oder 1-, 2-, 4-, oder 5-Imidazolyl-(C₁ -C₆)-Alkyl-Rest, wobei der (C₁ -C₆)-alkyl-Rest unsubstituiert oder ein- oder mehrfach gleich oder verschieden mit (C₁ -C₆)-Alkyl, Halogen oder Oxo (=O) substituiert sein kann und der 1-, 2-, 4-, oder 5-Imidazolyl -Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 3-, 4- oder 5-Pyrazolyl-Rest oder 1-, 3-, 4- oder 5-Pyrazolyl-(C₁ -C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 3-, 4- oder 5-Pyrazolyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 2,- 3,- 4,- oder 5-Pyrrolyl-Rest oder 1-, 2,- 3,- 4,- oder 5-Pyrrolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 2,- 3,- 4,- oder 5-Pyrrolyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 3-, oder 5-[1.2.4]-Triazolyl-Rest oder 1-, 3-, oder 5-[1.2.4]-Triazolyl-(C₁-C₆)-alkyl-Rest, wobei der (C₁-C₆)-alkyl-Rest unsubstituiert oder ein- oder mehrfach gleich oder verschieden mit Wasserstoff, (C₁-C₆)-Alkyl, Halogen oder Oxo (=O) substituiert sein kann und der 1-, 3-, oder 5-[1.2.4]-Triazolyl-Rest unsubstituiert oder ein- oder zweifach gleich oder verschieden mit (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 4-, oder 5-[1.2.3]-Triazolyl-Rest oder 1-, 4-, oder 5-[1.2.3]-Triazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 4-, oder 5-[1.2.3]-Triazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1- oder 5-[1*H*]-Tetrazolyl-Rest oder 1- oder 5-[1*H*]-Tetrazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1- oder 5-[1*H*]-Tetrazolyl-Rest unsubstituiert oder mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2- oder 5-[2*H*]-Tetrazolyl-Rest oder 2- oder 5-[2*H*]-Tetrazolyl-(C₁ -C₆)-alkyl-Rest, wobei der (C₁ -C₆)-alkyl-Rest unsubstituiert oder ein- oder mehrfach gleich oder verschieden mit (C₁ -C₆)-Alkyl, Halogen oder Oxo (=O) substituiert sein kann und der 2- oder 5-[2*H*]-Tetrazolyl-Rest unsubstituiert oder mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4-, oder 6-[1.3.5]-Triazinyl-Rest oder 2-, 4-, oder 6-[1.3.5]-Triazinyl-(C₁ -C₆)-alkyl-Rest, wobei der (C₁ -C₆)-alkyl-Rest unsubstituiert oder ein- oder mehrfach gleich oder verschieden mit Wasserstoff, (C₁ -C₆)-Alkyl, Halogen oder Oxo (=O) substituiert sein kann und der 2-, 4-, oder 6-[1.3.5]-Triazinyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 2-, 4-, oder 5-Oxazolyl-Rest oder 2-, 4-, oder 5-Oxazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=0) substituiert sein kann und der 2-, 4-, oder 5-Oxazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 3-, 4-, oder 5-Isoxazolyl-Rest oder 3-, 4-, oder 5-Isoxazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 3-, 4-, oder 5-Isoxazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann;
einen 1-, 2-, 3-, 4-, 5-, 6- oder 7-Indolyl-Rest oder 1-, 2-, 3-, 4-, 5-, 6- oder 7-Indolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 2-, 3-, 4-, 5-, 6- oder 7-Indolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, vorzugsweise Trifluormethyl, (C₆-C₁₀)-Aryl, oder (C₆-C₁₀)-Aryl-(C₁-C₆)-alkyl substituiert sein kann; bedeutet, sowie die Isomeren, insbesondere Tautomere, Diastereomere und Enantiomere, und den pharmazeitisch verträglichen Salzen, insbesondere Säureadditionssalze, davon.

Gemäß einer weiteren Ausführungsform werden Acridin-Derivate gemäß der allgemeinen Formel (1) bereitgestellt, dadurch gekennzeichnet, dass R, R₁, R₂, R₃, X, Z, P, Q, n und m die vorstehend genannten Bedeutungen besitzen und R4 für Phenyl steht, welches unsubstituiert oder mit ein bis fünf gleich oder verschiedenen (C₁-C₆)-Alkoxygruppen substituiert ist, wobei benachbarte Sauerstoffatome auch durch (C₁-C₂)-Alkylen-Gruppen verknüpft sein können.

Gemäß einer weiteren Ausführungsform werden Acridin-Derivate gemäß der allgemeinen Formel (1) bereitgestellt, dadurch gekennzeichnet, dass R, R₁, R₂, R₃, X, Z, P, Q, n und m die vorstehend genannten Bedeutungen besitzen und R₄ für 3,5-Dimethoxyphenyl steht.

Gemäß einer weiteren Ausführungsform werden Acridin-Derivate gemäß der allgemeinen Formel (1) bereitgestellt, dadurch gekennzeichnet, dass und R₄ die vorstehend genannten Bedeutungen hat, R, R₁, R₂, R₃ jeweils für ein Wasserstoffatom stehen, Z für ein Sauerstoffatom und X für ein Stickstoffatom, P und Q jeweils für zwei Wasserstoffatome (also- CH2-) stehen, m gleich Null ist und n für die ganze Zahl 2 steht.

Gemäß einer weiteren Ausführungsform werden Acridin-Derivate gemäß der allgemeinen Formel (1) bereitgestellt, dadurch gekennzeichnet, dass R, R₁, R₂ und R₃ jeweils für ein Wasserstoffatom stehen, Z für ein Sauerstoffatom und X für ein Stickstoffatom, P und Q jeweils für zwei Wasserstoffatome (also- CH2-) stehen, m gleich Null ist, n für die ganze Zahl 2 steht und R₄ für einen 3,5-Dimethoxyphenyl-Rest steht.

Gemäß einem weiteren Aspekt der Erfindung wird ein Verfahren zur Herstellung von Acridin-Derivaten nach einem der Ansprüche 1 bis 6, dadurch charakterisiert, dass eine Acridincarbonsäure der allgemeinen Formel (2), worin R, R1, R2, R3 die vorstehend genannten Bedeutungen besitzen, Z ein Sauerstoff- oder Schwefelatom bedeutet und Y für eine Abgangsgruppe wie Halogen, Hydroxy, (C1-C6)-Alkoxy vorzugsweise Methoxy und Ethoxy, -O-Tosyl, -O-Mesyl oder Imidazolyl steht, 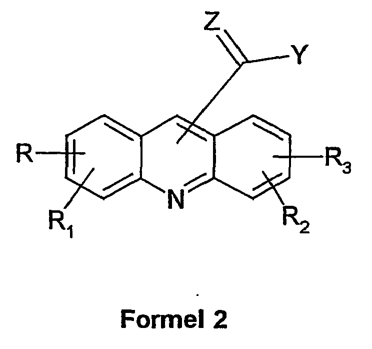 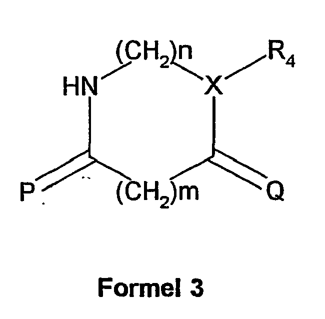 mit einem Amin der allgemeinen Formel (3), worin R4, P, Q, X, m und n die vorstehend genannten Bedeutungen besitzen, gegebenenfalls unter Verwendung von Verdünnungs- und Hilfsmitteln unter Bildung der gewünschten Acridin-Derivate umgesetzt wird.

### Syntheseweg:

Die Verbindungen der allgemeinen Formel 1 sind gemäß dem folgenden Schema 1 erhältlich:

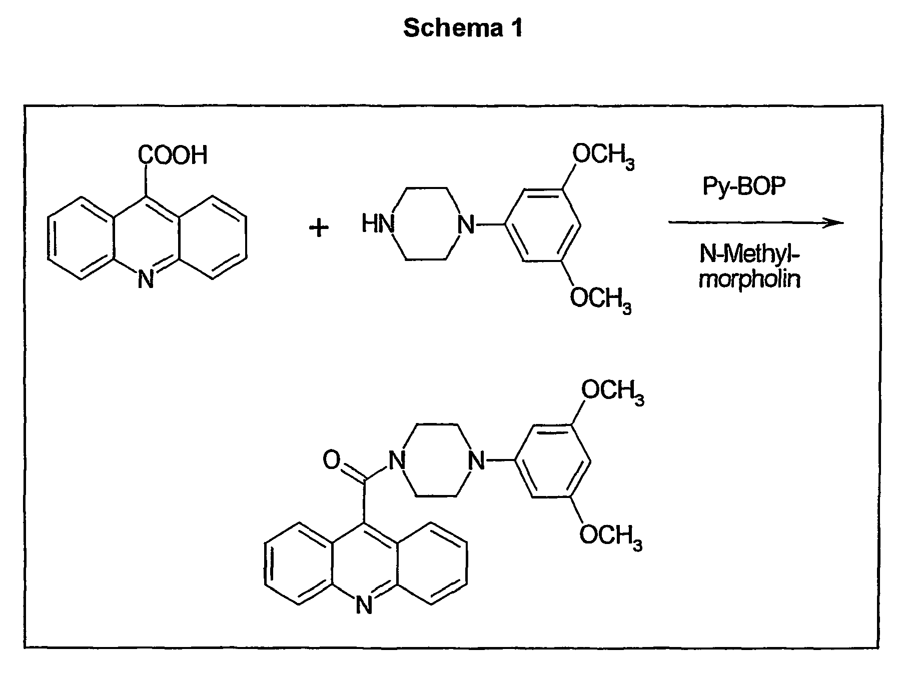

Die Ausgangsverbindungen (2) und (3) sind entweder im Handel erhältlich oder konnen nach an sich bekannten Verfahrensweisen hergestellt werden. Die Edukte (2) und (3) stellen wertvolle Zwischenverbindungen für die Herstellung der erfindungsgemäßen Acridin-Derivate der Formel (1) dar.

Die gegebenenfalls zu verwendenden Lösungs- und Hilfsmittel und anzuwendenden Reaktionsparameter wie Reaktionstemperatur und -dauer sind dem Fachmann aufgrund seines Fachwissens bekannt.

Die erfindungsgemäßen Acridin-Derivate gemäß der allgemeinen Formel (1) sind als Arzneimittel, insbesondere als Antitumormittel, zur Behandlung von Säugetieren, insbesondere dem Menschen, aber auch für Haustiere wie Pferde, Kühe, Hunde, Katzen, Hasen, Schafe, Geflügel und dergleichen geeignet.

Gemäß einem weiteren Aspekt der Erfindung wird ein Verfahren zur Bekämpfung von Tumoren in Säugetieren, insbesondere beim Menschen bereit gestellt, welches dadurch gekennzeichnet ist, dass mindestens ein Acridin-Derivat gemäß der allgemeinen Formel (1) einem Säugetier in einer für die Tumorbehandlung wirksamen Menge verabreicht wird. Die für die Behandlung zu verabreichende therapeutisch effektive Dosis des jeweiligen erfindungsgemäßen Acridin-Derivates richtet sich u.a. nach der Art und dem Stadium der Tumorerkrankung, dem Alter und Geschlecht des Patienten, der Art der Verabreichung und der Dauer der Behandlung. Die Verabreichung kann oral, rectal, buccal (z.B. sublingual), parenteral (z.B. subkutan, intramuskulär, intradermal oder intravenös), topisch oder transdermal erfolgen.

Gemäß einem weiteren Aspekt der Erfindung werden Arzneimittel zur Tumorbehandlung bereitgestellt, welche dadurch gekennzeichnet sind, dass sie als wirksamen Bestandteil mindestens ein Acridin-Derivat nach einem der Ansprüche 1 bis 5 oder einem pharmazeutisch verträglichen Salz davon, gegebenenfalls zusammen mit üblichen pharmazeutisch verträglichen Hilfs-, Zusatz- und Trägerstoffen enthalten. Es kann sich dabei um festen, halbfeste, flüssige oder Aerosol-Zubereitungen handeln. Geignete feste Zubereitungen sind beispielsweise Kapseln, Pulver, Granulate, Tabletten. Geeignete halbfeste Zubereitungen sind beispielsweise Salben, Cremes, Gele, Pasten, Suspensionen, Öl-in-Wasser- und Wasser-in-Öl-Emulsionen. Geeignete flüssige Zubereitungen sind beispielsweise sterile wäßrige Zubereitungen für die parenterale Verabreichung, die isoton mit dem Blut des Patienten sind.

Die Erfindung soll anhand des nachfolgenden Beispiels näher erläutert werden, ohne darauf beschränkt zu sein.

### Ausführungsbeispiel

### 1-(3,5-Dimethoxyphenyl)-4-(9-acridinyl-carbonyl) piperazin (D-43411)

8g (35,84 mMol) Acridin-9-carbonsäure wurden unter Rühren in 300 ml DMF vorgelegt. Zu dem Gemisch gab man unter weiterem Rühren nacheinander 5,79g (57,34 mMol) N-Methylmorpholin, sodann eine Lösung von 24,24g (46,59 mMol) Py-BOP (1-Benzotriazolyltripyrrolidinophosphoniumhexafluorphosphat) und 7,96 (35,81 mMol) 1-(3,5-Dimethoxyphenyl) piperazin in 50 ml DMF. Es wurde 12 Stunden bei Raumtemperatur gerührt, das DMF im Vakuum abdestiliert und der Rückstand über eine Kieselgelsäule (Kieselgel 60, Fa. Merck AG, Darmstadt) unter Anwendung des Elutionsmittels Dichlormethan/Methanol (95:5 V/V) gereinigt.
Ausbeute: 12,9g (84,2% d.Th.)
Fp.: 172-175°C

### 1. Anti-proliferative Wirkung an verschiedenen Tumor Zelllinien

Die Substanz D-43411 wurde in einem Proliferationstest an etablierten Tumorzelllinien auf ihre anti-proliferative Aktivität hin untersucht. Der verwendete Test bestimmt die zelluläre Dehydrogenase Aktivität und ermöglicht eine Bestimmung der Zellvitalität und indirekt der Zellzahl. Bei den verwendeten Zelllinien handelt es sich um die humane Cervixkarzinom Zelllinie KB / HeLa (ATCC CCL17), die murine lymphozytäre Leukämie L1210 (ATCC CCL-219), die humane Brustadenokarzinomlinie MCF7 (ATCC HTB22) und die ovariale Adenokarzinomlinie SKOV-3 (ATCC HTB77). Es handelt sich hierbei um sehr gut charakterisierte, etablierte Zelllinien, die von ATCC erhalten und in Kultur genommen wurden.

Die in Tab. 1 gezeigten Ergebnisse belegen eine sehr potente anti-proliferative Wirkung von D-43411 an den Zelllinien SKOV-3, L-1210 und HeLa/KB. Aufgrund der Besonderheit des langsamen Wachstums der MCF7 Linie ist die Wirkung von D-43411 im Versuchszeitraum von 48h nur gering (18% Hemmung bei 3.16 µg/ml; daher Angabe >3.16).

**Tab. 1 Zytotoxizität an Tumorzelllinien in-vitro (Werte bestimmt aus 5 Substanzkonzentrationen)**

| | | | XTT- Assay IC₅₀ [µg/ml] | | | |
|---|---|---|---|---|---|---|
| D-Nummer | Struktur | MG | SKOV-3 | L1210 | KB/HeLa | MCF7 |
| D-43411 | 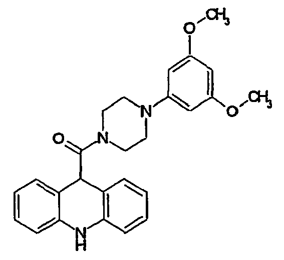 | 429 | <0.0003 | <0.0003 | <0.0003 | >3.16 |

### 2. Methode

### XTT-Test auf zelluläre Dehydrogenase-Aktivität

Die adherent wachsenden TumorZelllinen HeLa/KB, SKOV-3 und MCF7 sowie die in Suspension wachsende L1210 Leukämielinie wurden unter Standardbedingungen im Begasungsbrutschrank bei 37°C, 5% CO₂ und 95% Luftfeuchtigkeit kultiviert. Am Versuchstag 1 werden die adherenten Zellen mit Trypsin / EDTA abgelöst und durch Zentrifugation pelletiert. Nachfolgend wird das Zellpellet im RPMI Kulturmedium in der entsprechenden Zellzahl resuspendiert und in eine 96-well Mikrotiterplatte umgesetzt. Die Platten werden dann über Nacht im Begasungsbrutschrank kultiviert.

Die Testsubstanzen werden als Stammlösungen in DMSO angesetzt und am Versuchstag 2 mit Kulturmedium in den entsprechenden Konzentrationen verdünnt. Die Substanzen in Kulturmedium werden dann zu den Zellen gegeben und für 45h im Begasungsbrutschrank inkubiert. Als Kontrolle dienen Zellen, die nicht mit Testsubstanz behandelt werden.

Für das XTT-Assay werden 1mg/ml XTT (Natrium 3'-[1-(phenylaminocarbonyl)-3,4-tetrazolium]-bis(4-methoxy-6-nitro)benzensulfonsäure) in RPMI-1640 Medium ohne Phenolrot gelöst. Zusätzlich wird eine 0,383 mg/ml PMS (N-Methyl Dibenzopyrazine Methylsulfat) Lösung in Phosphat-gepufferter Salzlösung (PBS) hergestellt. Am Versuchstag 4 wird auf die Zellplatten, die inzwischen 45 h mit den Testsubstanzen inkubiert wurden, 75µl/well XTT-PMS-Mischung pipettiert. Dazu wird kurz vor Gebrauch die XTT-Lösung mit der PMS-Lösung im Verhältnis 50:1 (Vol:Vol) gemischt. Anschließend werden die Zellplatten im Begasungsbrutschrank für weitere 3h inkubiert und im Photometer die optische Dichte (OD₄₉₀ₙₘ) bestimmt.
Mittels der bestimmten OD₄₉₀ₙₘ wird die prozentuale Hemmung relativ zur Kontrolle berechnet. Die anti-proliferative Wirkung wird mittels einer Regressionsanalyse abgeschätzt.

### Beispiel I

### Tablette mit 50 mg Wirkstoff

### Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| Summe: | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt.

### Beispiel II

### Kapsel mit 50 mg Wirkstoff

### Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| Summe: | 160,0 mg |

### Herstellung:

(1) wird mit (3) verrieben. Diese Verreibung wird der Mischung aus (2) und (4) unter intensiver Mischung zugegeben. Diese Pulvermischung wird auf einer Kapselabfüllmaschine in Hartgelatine-Steckkapseln Größe 3 abgefüllt.

## Patentansprüche

1. Acridin-Derivate gemäß der allgemeinen Formel 1 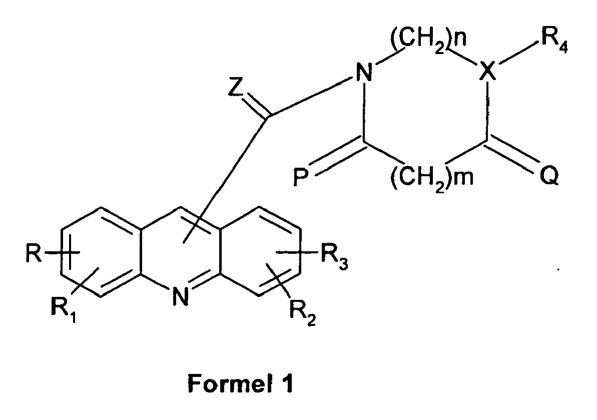 worin
R, R₁, R₂, R₃ wahlweise an den Acridin-Kohlenstoffatomen C₁ bis C₉ gebunden sein können, gleich oder verschieden sind und unabhängig voneinander Wasserstoff, geradkettiges oder verzweigtes (C₁-C₈)-Alkyl, (C₃-C₇)-Cycloalkyl, geradkettigtes oder verzweigtes (C₁-C₈)-Alkylcarbonyl, geradkettiges oder verzweigtes (C₁-C₈)-Alkoxy, Halogen, Aryl-(C₁-C₈)-alkoxy, Nitro, Amino, Mono-(C₁-C₄)-Alkylamino, Di-(C₁-C₄)-Alkylamino, (C₁-C₈)-Alkoxycarbonyl-amino, (C₁-C₆)-Alkoxycarbonylamino-(C₁-C₈)-alkyl, Cyano, geradkettiges oder verzweigtes Cyano-(C₁-C₆)-alkyl, Carboxy, (C₁-C₈)-Alkoxycarbonyl, mit einem oder mehreren Fluoratomen substituiertes (C₁-C₄)-Alkyl, Carboxy-(C₁-C₈)-alkyl oder (C₁-C₈)-Alkoxycarbonyl-(C₁-C₆)-alkyl, (C₂-C₆)-Alkenyl, (C₂-C₆)-Alkinyl, geradkettiges oder verzweigtes Cyano-(C₁-C₆)-alkyl, Aryl, wobei der Arylrest unsubstituiert oder ein-oder mehrfach gleich oder verschieden mit Halogen, geradkettigem oder verzweigtem (C₁-C₈)-Alkyl, (C₃-C₇)-Cycloalkyl, Carboxy, geradkettigem oder verzweigtem (C₁-C₈)-Alkoxycarbonyl, mit Trifluormethyl, Hydroxy, geradkettigem oder verzweigtem (C₁-C₈)-Alkoxy, Benzyloxy, Nitro, Amino, Mono-(C₁-C₄)-Alkylamino, Di-(C₁-C₄)-Alkylamino, Cyano, geradkettigem oder verzweigtem Cyano-(C₁-C₆)-alkyl substituiert sein kann, bedeuten,
Z Sauerstoff oder Schwefel ist, wobei der am Acridin-Heterocyclus substituierte Rest 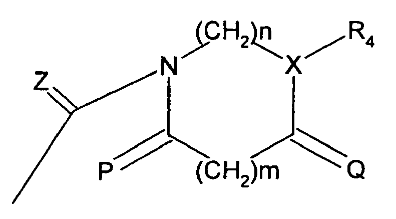 an den C-Atomen C₁-C₉ des Acridin-Ringgerüstes gebunden sein kann;
P, Q unabhängig voneinander für Sauerstoff oder jeweils für zwei Wasserstoffatome stehen;
X Stickstoff oder C-R₅ ist, wobei R₅ für Wasserstoff oder (C₁-C₆)-Alkyl steht
n,m unabhängig voneinander eine ganze Zahl zwischen 0-3 bedeuten, mit der Maßgabe, dass im Falle n=0 X eine CR₅R₆-Gruppe, wobei R₅ und R₆ unabhängig voneinander für Wasserstoff oder (C₁-C₆)-Alkyl stehen, bedeutet und an dem der C=Z-Gruppe benachbarten Stickstoff-Atom ein Wasserstoff-Atom oder eine (C₁-C₆)-Alkylgruppe substituiert ist,
R₄ einen geradkettigen oder verzweigten (C₁-C₂₀)-Alkyl-Rest, welcher gesättigt oder mit ein bis drei Doppel- und/oder Dreifachbindungen ungesättigt sein kann und welcher unsubstituiert oder wahlweise an dem gleichen oder verschiedenen C-Atomen mit ein, zwei oder mehreren Aryl, Heteroaryl, Halogen, Cyano, (C1-C6)-Alkoxycarbonylamino, (C1-C6)-Alkoxy, Amino, Mono-(C₁-C₄) Alkylamino oder Di- (C₁-C₄)-Alkylamino substituiert sein kann; einen (C₆-C₁₄)-Aryl-Rest, (C₆-C₁₄)-Aryl-(C₁-C₄)-alkyl-Rest oder einen ein oder mehrere Heteroatome ausgewählt aus der Gruppe N, O und S enthaltenden (C₂-C₁₀)-Heteroaryl- oder (C₂-C₁₀)-Heteroaryl-(C₁-C₄)-alkyl-Rest, wobei der
(C₁ -C₄)-alkyl-Rest unsubstituiert oder ein- mehrfach gleich oder verschieden mit (C₁ -C₆)-Alkyl, Halogen oder Oxo (=O) substituiert sein kann und der (C₆-C₁₄)-Aryl- oder (C₂-C₁₀)-Heteroaryl-Rest unsubstituiert oder ein- oder mehrfach gleich oder verschieden mit geradkettigem oder verzweigtem (C₁-C₈)-Alkyl, (C₃-C₇)-Cycloalkyl, Halogen, Cyano, (C1-C6)-Alkoxycarbonylamino, (C1-C6)-Alkoxy, Carboxy, (C₁-C₈)-Alkoxycarbonyl, mit einem oder mehreren Fluoratomen substituierten geradkettigem oder verzweigtem (C₁-C₆)-Alkyl, Hydroxy, geradkettigem oder verzweigtem (C₁-C₈)-Alkoxy, wobei benachbarte Sauerstoffatome auch durch (C₁-C₂)-Alkylen-Gruppen verknüpft sein können, Benzyloxy, Nitro, Amino, Mono-(C₁-C₄)-Alkylamino, Di-(C₁-C₄)-Alkylamino, Aryl, das seinerseits unsubstituiert oder ein- oder mehrfach gleich oder verschieden mit geradkettigem oder verzweigtem (C₁-C₈)-Alkyl, (C₃-C₇)-Cycloalkyl, Carboxy, geradkettigem oder verzweigtem (C₁-C₈)-Alkoxycarbonyl, mit Trifluormethyl, Hydroxy, geradkettigem oder verzweigtem (C₁-C₈)-Alkoxy, Benzyloxy, Nitro, Amino, Mono-(C₁-C₄) Alkylamino, Di- (C₁-C₄)-Alkylamino, Cyano, geradkettigem oder verzweigtem Cyano-(C₁-C₆)-alkyl substituiert ist, substituiert sein kann;
sowie deren Struktur- und Stereoisomeren, insbesondere Tautomere, Diastereomere und Enantiomere, und deren pharmazeutisch verträglichen Salzen.

2. Acridin-Derivate gemäß der allgemeinen Formel 1 nach Anspruch 1, **dadurch gekennzeichnet, dass** R, R1, R2, R3, X, Z, P, Q, n und m die in Anspruch 1 genannten Bedeutungen besitzen und
R4 einen geradkettigen oder verzweigten (C₁-C₂₀)-Alkyl-Rest, welcher gesättigt oder mit ein bis drei Doppel- und/oder Dreifachbindungen ungesättigt sein kann und welcher unsubstituiert oder wahlweise an dem gleichen oder verschiedenen C-Atomen mit ein, zwei oder mehreren Aryl, Heteroaryl, Halogen, (C1-C6)-Alkoxy, Amino, Mono-(C₁-C₄) Alkylamino oder Di- (C₁-C₄)-Alkylamino substituiert sein kann; einen Phenylring oder einen Naphthylring, die unsubstituiert oder ein- oder mehrfach gleich oder verschieden mit geradkettigem oder verzweigtem (C₁-C₈)-Alkyl, (C₃-C₇)-Cycloalkyl, Halogen, Cyano, (C₁-C₆)-Alkoxycarbonylamino, (C₁-C₆)-Alkoxy, Carboxy, (C₁-C₈)-Alkoxycarbonyl, mit einem oder mehreren Fluoratomen substituierten geradkettigem oder verzweigtem (C₁-C₆)-Alkyl, Hydroxy, geradkettigem oder verzweigtem (C₁-C₈)-Alkoxy, Benzyloxy, Nitro, Amino, Mono-(C₁-C₄)-Alkylamino, Di-(C₁-C₄)-Alkylamino, Aryl, das seinerseits unsubstituiert oder ein- oder mehrfach gleich oder verschieden mit geradkettigem oder verzweigtem (C₁-C₈)-Alkyl, (C₃-C₇)-Cycloalkyl, Carboxy, geradkettigem oder verzweigtem (C₁-C₈)-Alkoxycarbonyl, mit Trifluormethyl, Hydroxy, geradkettigem oder verzweigtem (C₁-C₈)-Alkoxy, Benzyloxy, Nitro, Amino, Mono-(C₁-C₄) Alkylamino, Di- (C₁-C₄)-Alkylamino, Cyano, geradkettigem oder verzweigtem Cyano-(C₁-C₆)-alkyl substituiert ist, substituiert sein können,
einen 2-, 4-, 5- oder 6-Pyrimidinyl-Rest oder 2-, 4-, 5- oder 6-Pyrimidinyl-(C₁-C₄)-alkyl-Rest, wobei der (C₁ -C₄)-alkyl-Rest unsubstituiert oder ein- mehrfach gleich oder verschieden mit (C₁ -C₆)-Alkyl, Halogen oder Oxo (=O) substituiert sein kann und der 2-, 4-, 5- oder 6-Pyrimidinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 3-, 4-, 5-, oder 6-Pyridazinyl-Rest oder 3-, 4-, 5-, oder 6-Pyridazinyl-(C₁-C₄)-alkyl-Rest, wobei der (C₁-C₄)-alkyl-Rest unsubstituiert oder ein- mehrfach gleich oder verschieden mit (C₁-C₆)-Alkyl, Halogen oder Oxo (=O) substituiert sein kann und der 3-, 4-, 5-, oder 6-Pyridazinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, (C₆-C₁₀)-Aryl, oder (C₆-C₁₀)-Aryl-(C₁-C₆)-alkyl substituiert sein kann;
einen 2-, 3-, 5,- oder 6-Pyrazinyl-Rest oder 2-, 3-, 5,- oder 6-Pyrazinyl-(C₁-C₄)-alkyl-Rest, wobei der (C₁ -C₄)-alkyl-Rest unsubstituiert oder ein- mehrfach gleich oder verschieden mit (C₁ -C₆)-Alkyl, Halogen oder Oxo (=O) substituiert sein kann und der 2-, 3-, 5,- oder 6-Pyrazinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, (C₆-C₁₀)-Aryl, oder (C₆-C₁₀)-Aryl-(C₁-C₆)-alkyl substituiert sein kann;
einen 3-, 4-, 5-, 6-, 7-, oder 8-Cinnolinyl-Rest oder 3-, 4-, 5-, 6-, 7-, oder 8-Cinnolinyl-(C₁-C₄)-alkyl-Rest, wobei der (C₁-C₄)-alkyl-Rest unsubstituiert oder ein- mehrfach gleich oder verschieden mit (C₁-C₆)-Alkyl, Halogen oder Oxo (=O) substituiert sein kann und der 3-, 4-, 5-, 6-, 7-, oder 8-Cinnolinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, (C₆-C₁₀)-Aryl, oder (C₆-C₁₀)-Aryl-(C₁-C₆)-alkyl substituiert sein kann;
einen 2-, 4-, 5-, 6-, 7-, oder 8-Chinazolinyl-Rest oder 2-, 4-, 5-, 6-, 7-, oder 8-Chinazolinyl-(C₁ -C₄)-alkyl-Rest, wobei der (C₁ -C₄)-alkyl-Rest unsubstituiert oder ein- mehrfach gleich oder verschieden mit Wasserstoff, (C₁ -C₆)-Alkyl, Halogen oder Oxo (=O) substituiert sein kann und der oder 2-, 4-, 5-, 6-, 7-, oder 8-Chinazolinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 2-, 3-, 5-, 6-, 7-, oder 8-Chinoxalinyl-Rest 2-, 3-, 5-, 6-, 7-, oder 8-Chinoxalinyl-(C₁-C₄)-alkyl-Rest, wobei der (C₁-C₄)-alkyl-Rest unsubstituiert oder ein- mehrfach gleich oder verschieden mit (C₁-C₆)-Alkyl, Halogen oder Oxo (=O) substituiert sein kann und der oder 2-, 3-, 5-, 6-, 7-, oder 8-Chinoxalinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, (C₆-C₁₀)-Aryl, oder (C₆-C₁₀)-Aryl-(C₁-C₆)-alkyl substituiert sein kann;
einen 1-, 4-, 5-, 6-, 7-, oder 8-Phthalazinyl-Rest oder 1-, 4-, 5-, 6-, 7-, oder 8-Phthalazinyl-(C₁ -C₄)-alkyl-Rest, wobei der (C₁ -C₄)-alkyl-Rest unsubstituiert oder ein- mehrfach gleich oder verschieden mit (C₁ -C₆)-Alkyl, Halogen oder Oxo (=O) substituiert sein kann und der oder 1-, 4-, 5-, 6-, 7-, oder 8-Phthalazinyl-Rest unsubstituiert oder ein- bis fünffach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, (C₆-C₁₀)-Aryl, oder (C₆-C₁₀)-Aryl-(C₁-C₆)-alkyl substituiert sein kann;
einen 2-, 3-, 4-, 5-, 6-, 7- oder 8-Chinolyl-Rest oder 2-, 3-, 4-, 5-, 6-, 7- oder 8-Chinolyl-(C₁ -C₄)-alkyl-Rest, wobei der (C₁ -C₄)-alkyl-Rest unsubstituiert oder ein- oder mehrfach gleich oder verschieden mit (C₁ -C₆)-Alkyl, Halogen oder Oxo (=O) substituiert sein kann und der 2-, 3-, 4-, 5-, 6-, 7- oder 8-Chinolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, (C₆-C₁₀)-Aryl, oder (C₆-C₁₀)-Aryl-(C₁-C₆)-alkyl substituiert sein kann;
einen 1-, 3-, 4-, 5-, 6-, 7- oder 8-Isochinolyl- oder 1-, 3-, 4-, 5-, 6-, 7- oder 8-Isochinolyl-(C₁-C₄)-alkyl-Rest, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der 1-, 3-, 4-, 5-, 6-, 7- oder 8-Isochinolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 2-, 6-, 8- oder 9-[9*H*]-Purinyl-Rest oder 2-, 6-, 8- oder 9-[9*H*]-Purinyl-(C₁-C₄)-alkyl-Rest, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der 2-, 6-, 8- oder 9-[9*H*]-Purinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 2-, 6-, 7- oder 8-[7*H*]-Purinyl-Rest oder 2-, 6-, 7- oder 8-[7*H*]-Purinyl-(C₁-C₄)-alkyl-Rest, wobei der (C₁-C₄)-alkyl-Rest unsubstituiert oder ein- oder mehrfach gleich oder verschieden mit (C₁-C₆)-Alkyl, Halogen oder Oxo (=O) substituiert sein kann und der 2-, 6-, 7- oder 8-[7*H*]-Purinyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 1-, 2-, 3-, 4-, 5-, 6-, 7-, 8- oder 9-Acridinyl- oder 1-, 2-, 3-, 4-, 5-, 6-, 7-, 8-oder 9-Acridinyl-(C₁-C₄)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 2-, 3-, 4-, 5-, 6-, 7-, 8- oder 9-Acridinyl-Rest unsubstituiert oder ein- bis achtfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 1-, 2-, 3-, 4-, 5-, 6-, 7-, 8- oder 9-Phenanthridinyl- oder 1-, 2-, 3-, 4-, 5-, 6-, 7-, 8- oder 9- Phenanthridinyl-(C₁-C₆)-alkyl-Rest, wobei der (C₁-C₆)-alkylRest unsubstituiert oder ein- oder mehrfach gleich oder verschieden mit Wasserstoff, (C₁-C₆)-Alkyl, Halogen oder Oxo (=O) substituiert sein kann und der 1-, 2-, 3-, 4-, 5-, 6-, 7-, 8- oder 9-Phenanthridinyl-Rest unsubstituiert oder ein- bis achtfach gleich oder verschieden mit (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, (C₆-C₁₀)-Aryl-(C₁-C₆)-alkoxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 2-, 3-, 4-, 5,- oder 6-Pyridyl-Rest, wobei der 2-, 3-, 4-, 5,- oder 6-Pyridinyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 2-, 3-, 4-, 5,- oder 6-Pyridyl-(C₁-C₆)-alkyl-Rest, wobei der (C₁-C₆)-alkylRest unsubstituiert oder ein- oder mehrfach gleich oder verschieden mit (C₁-C₆)-Alkyl, Halogen oder Oxo (=O) substituiert sein kann und der 2-, 3-, 4-, 5,- oder 6-Pyridinyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 2-, 3-, 4,- oder 5-Thienyl-Rest oder 2-, 3-, 4,- oder 5-Thienyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2-, 3-, 4,- oder 5-Thienyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 2-, 4-, oder 5-Thiazolyl-Rest oder 2-, 4-, oder 5-Thiazolyl-(C₁-C₆)-alkylRest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2-, 4-, oder 5-Thiazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 3-, 4-, oder 5-Isothiazolyl-Rest oder 3-, 4-, oder 5-Isothiazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 3-, 4-, oder 5-Isothiazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 2-, 4-, 5-, 6-, oder 7-Benzthiazolyl-Rest oder 2-, 4-, 5-, 6-, oder 7-Benzthiazolyl (C₁ -C₆)-alkyl-Rest, wobei der (C₁ -C₆)-alkyl-Rest unsubstituiert oder ein- oder mehrfach gleich oder verschieden mit (C₁ -C₆)-Alkyl, Halogen oder Oxo (=O) substituiert sein kann und der 2-, 4-, 5-, 6-, oder 7-Benzthiazolyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, (C₆-C₁₀)-Aryl, oder (C₆-C₁₀)-Aryl-(C₁-C₆)-alkyl substituiert sein kann;
einen 1-, 2-, 4-, oder 5-Imidazolyl-Rest oder 1-, 2-, 4-, oder 5-Imidazolyl-(C₁ -C₆)-alkyl-Rest, wobei der (C₁ -C₆)-alkyl-Rest unsubstituiert oder ein- oder mehrfach gleich oder verschieden mit (C₁ -C₆)-Alkyl, Halogen oder Oxo (=O) substituiert sein kann und der 1-, 2-, 4-, oder 5-Imidazolyl -Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 1-, 3-, 4- oder 5-Pyrazolyl-Rest oder 1-, 3-, 4- oder 5-Pyrazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 3-, 4- oder 5-Pyrazolyl-Rest unsubstituiert oder ein- bis dreifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 1-, 2,- 3,- 4,- oder 5-Pyrrolyl-Rest oder 1-, 2,- 3,- 4,- oder 5-Pyrrolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 2,- 3,- 4,- oder 5-Pyrrolyl-Rest unsubstituiert oder ein- bis vierfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 1-, 3-, oder 5-[1.2.4]-Triazolyl-Rest oder 1-, 3-, oder 5-[1.2.4]-Triazolyl-(C₁-C₆)-alkyl-Rest, wobei der (C₁-C₆)-alkyl-Rest unsubstituiert oder ein- oder mehrfach gleich oder verschieden mit Wasserstoff, (C₁-C₆)-Alkyl, Halogen oder Oxo (=O) substituiert sein kann und der 1-, 3-, oder 5-[1.2.4]-Triazolyl-Rest unsubstituiert oder ein- oder zweifach gleich oder verschieden mit (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 1-, 4-, oder 5-[1.2.3]-Triazolyl-Rest oder 1-, 4-, oder 5-[1.2.3]-Triazolyl-(C₁ -C₆)-alkyl-Rest, wobei der (C₁ -C₆)-alkyl-Rest unsubstituiert oder ein- oder mehrfach gleich oder verschieden mit (C₁ -C₆)-Alkyl; Halogen oder Oxo (=O) substituiert sein kann und der 1-, 4-, oder 5-[1.2.3]-Triazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, (C₆-C₁₀)-Aryl, oder (C₆-C₁₀)-Aryl-(C₁-C₆)-alkyl substituiert sein kann;
einen 1- oder 5-[1*H*]-Tetrazolyl-Rest oder 1- oder 5-[1*H*]-Tetrazolyl-(C₁ -C₆)-alkyl-Rest, wobei der (C₁ -C₆)-alkyl-Rest unsubstituiert oder ein- oder mehrfach gleich oder verschieden mit (C₁ -C₆)-Alkyl, Halogen oder Oxo (=O) substituiert sein kann und der 1- oder 5-[1*H*]-Tetrazolyl-Rest unsubstituiert oder mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁ -C₆)-Alkyl, (C₆-C₁₀)-Aryl, oder (C₆-C₁₀)-Aryl-(C₁-C₆)-alkyl substituiert sein kann;
einen 2- oder 5-[2*H*]-Tetrazolyl-Rest oder 2- oder 5-[2*H*]-Tetrazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2- oder 5-[2*H*]-Tetrazolyl-Rest unsubstituiert oder mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 2-, 4-, oder 6-[1.3.5]-Triazinyl-Rest oder 2-, 4-, oder 6-[1.3.5]-Triazinyl-(C₁-C₆)-alkyl-Rest, wobei der (C₁-C₆)-alkyl-Rest unsubstituiert oder ein- oder mehrfach gleich oder verschieden mit Wasserstoff, (C₁-C₆)-Alkyl, Halogen oder Oxo (=O) substituiert sein kann und der 2-, 4-, oder 6-[1.3.5]-Triazinyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 2-, 4-, oder 5-Oxazolyl-Rest oder 2-, 4-, oder 5-Oxazolyl-(C₁-C₆)-alkylRest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 2-, 4-, oder 5-Oxazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 3-, 4-, oder 5-Isoxazolyl-Rest oder 3-, 4-, oder 5-Isoxazolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 3-, 4-, oder 5-Isoxazolyl-Rest unsubstituiert oder ein- oder zweifach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann;
einen 1-, 2-, 3-, 4-, 5-, 6- oder 7-Indolyl-Rest oder 1-, 2-, 3-, 4-, 5-, 6- oder 7-Indolyl-(C₁-C₆)-alkyl-Rest, wobei der (C₁-C₆)-alkyl-Rest unsubstituiert oder ein- oder mehrfach gleich oder verschieden mit (C₁-C₆)-Alkyl, Halogen oder Oxo (=O) substituiert sein kann und der 1-, 2-, 3-, 4-, 5-, 6- oder 7-Indolyl-Rest unsubstituiert oder ein- bis sechsfach gleich oder verschieden mit Wasserstoff, (C₁-C₆)-Alkyl, Halogen, Nitro, Amino, Mono-(C₁-C₆)-Alkylamino, Di-(C₁-C₆)-Alkylamino, Hydroxy, (C₁-C₆)-Alkoxy, Benzyloxy, Carboxy, (C₁-C₆)-Alkoxycarbonyl, (C₁-C₆)-Alkoxycarbonylamino oder ein- oder mehrfach mit Fluor substituiertes (C₁-C₆)-Alkyl, (C₆-C₁₀)-Aryl, oder (C₆-C₁₀)-Aryl-(C₁-C₆)-alkyl substituiert sein kann; bedeutet.

3. Acridin-Derivate nach Anspruch 1 oder 2, **dadurch gekennzeichnet, dass** R, R₁, R₂, R₃, X, Z, P, Q, n und m die vorstehend genannten Bedeutungen besitzen und R4 für Phenyl steht, welches unsubstituiert oder mit ein bis fünf gleich oder verschiedenen (C₁-C₆)-Alkoxygruppen substituiert ist, wobei benachbarte Sauerstoffatome auch durch (C₁-C₂)-Alkylen-Gruppen verknüpft sein können.

4. Acridin-Derivate nach einem der Ansprüche 1 bis 3, **dadurch gekennzeichnet, dass** R, R₁, R₂, R₃, P, Q, X, Z, n und m die vorstehend genannten Bedeutungen besitzen und R₄ für 3,5-Dimethoxyphenyl steht.

5. Acridin-Derivate nach einem der vorstehenden Ansprüche, **dadurch gekennzeichnet, dass** R₄ die vorstehend genannten Bedeutungen besitzt, R, R₁, R₂ und R₃ jeweils für ein Wasserstoffatom stehen, Z für ein Sauerstoffatom und X für ein Stickstoffatom, P und Q jeweils für zwei Wasserstoffatome stehen und m gleich Null ist und n für die ganze Zahl 2 steht.

6. Acridin-Derivate nach einem der vorstehenden Ansprüche, **dadurch gekennzeichnet, dass** R, R₁, R₂, R₃ jeweils für ein Wasserstoffatom stehen, Z für ein Sauerstoffatom und X für ein Stickstoffatom, P und Q jeweils für zwei Wasserstoffatome stehen und m gleich Null ist und n für die ganze Zahl 2 steht und R₄ für 3,5-Dimethoxyphenyl steht.

7. Acridin-Derivate nach einem der Ansprüche 1 bis 6 zur Verwendung als Arzneimittel.

8. Verwendung der Acridin-Derivate nach einem der Ansprüche 1 bis 6 zur Herstellung eines Arzneimittel zur Behandlung von Tumoren in Säugetieren.

9. Verfahren zur Herstellung von Acridin-Derivaten nach einem der Ansprüche 1 bis 6, **dadurch gekennzeichnet, dass** eine Acridincarbonsäure der allgemeinen Formel (2), worin R, R1, R2, R3 die vorstehend genannten Bedeutungen besitzen, Z ein Sauerstoff- oder Schwefelatom bedeutet und Y für eine Abgangsgruppe wie Halogen, Hydroxy, (C1-C6)-Alkoxy, -O-Tosyl, -O-Mesyl oder Imidazolyl steht, 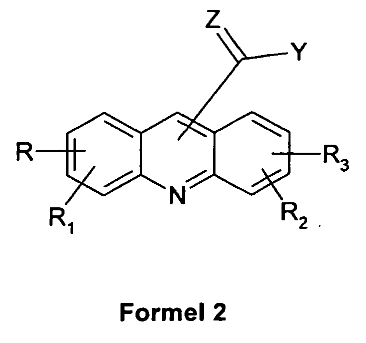 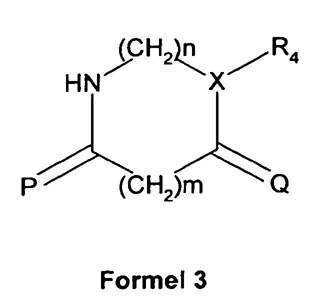 mit einem Amin der allgemeinen Formel (3), worin R4, X, P, Q, m und n die vorstehend genannten Bedeutungen besitzen, gegebenenfalls unter Verwendung von Verdünnungs- und Hilfsmitteln unter Bildung des gewünschten Acridin-Derivates umgesetzt wird.

10. Arzneimittel, **dadurch gekennzeichnet, dass** es als wirksamen Bestandteil mindestens ein Acridin-Derivat nach einem der Ansprüche 1 bis 6 gegebenenfalls zusammen mit üblichen pharmazeutisch verträglichen Hilfs-, Zusatz- und Trägersstoffen enthält.

## Claims

1. Acridine derivatives according to the formula 1 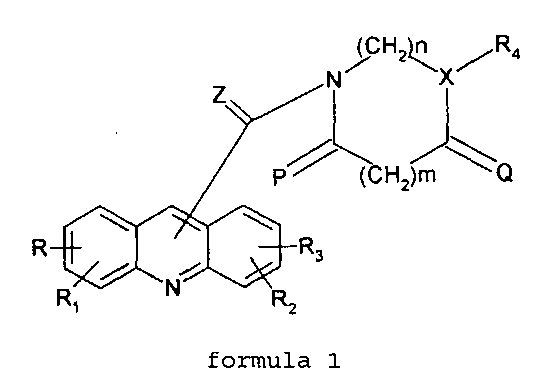 in which
R, R₁, R₂, R₃ can be attached to any of the acridine carbon atoms C₁ to C₉, are identical or different and independently of one another denote hydrogen, straight-chain or branched (C₁-C₈)-alkyl, (C₃-C₇)-cycloalkyl, straight-chain or branched (C₁-C₈)-alkylcarbonyl, straight-chain or branched (C₁-C₈)-alkoxy, halogen, aryl-(C₁-C₈)-alkoxy, nitro, amino, mono-(C₁-C₄)-alkylamino, di-(C₁-C₄)-alkylamino, (C₁-C₈)-alkoxycarbonylamino, (C₁-C₆) -alkoxycarbonylamino- (C₁-C₈) -alkyl, cyano, straight-chain or branched cyano-(C₁-C₆)-alkyl, carboxyl, (C₁-C₈)-alkoxycarbonyl, (C₁-C₄)-alkyl which is substituted by one or more fluorine atoms, carboxy-(C₁-C₈)-alkyl or (C₁-C₈) -alkoxycarbonyl- (C₁-C₆) -alkyl, (C₂-C₆)-alkenyl, (C₂-C₆)-alkynyl, straight-chain or branched cyano-(C₁-C₆)-alkyl, aryl, where the aryl radical may be unsubstituted or mono- or polysubstituted by identical or different substituents from the group consisting of halogen, straight-chain or branched (C₁-C₈)-alkyl, (C₃-C₇)-cycloalkyl, carboxyl, straight-chain or branched (C₁-C₈)-alkoxycarbonyl, by trifluoromethyl, hydroxyl, straight-chain or branched (C₁-C₈)-alkoxy, benzyloxy, nitro, amino, mono-(C₁-C₄)-alkylamino, di-(C₁-C₄)-alkylamino, cyano, straight-chain or branched cyano-(C₁-C₆)-alkyl,
Z is oxygen or sulfur, where the radical 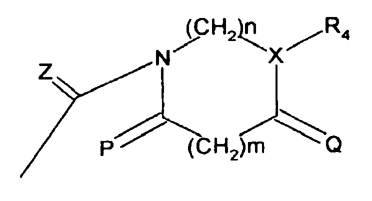 substituted on the acridine heterocycle may be attached to C atoms C₁-C₉ of the acridine ring skeleton;
P,Q independently of one another represent oxygen or in each case two hydrogen atoms;
X is nitrogen or C-R₅, where R₅ represents hydrogen or (C₁-C₆)-alkyl;
n,m independently of one another denotes an integer between 0-3, with the proviso that in the case n = 0, X denotes a CR₅R₆ group where R₅ and R₆ independently of one another represent hydrogen or (C₁-C₆)-alkyl and that the nitrogen atom adjacent to the C=Z group is substituted by a hydrogen atom or a (C-C₆)-alkyl group;
R₄ a straight-chain or branched (C₁-C₂₀)-alkyl radical which may be saturated or unsaturated, with one to three double and/or triple bonds, and which may be unsubstituted or may optionally be substituted at the same or different C atoms by one, two or more aryl, heteroaryl, halogen, cyano, (C1-C6)-alkoxycarbonylamino, (C1-C6)-alkoxy, amino, mono-(C₁-C₄)-alkylamino or di-(C₁-C₄)-alkylamino; a (C₆-C₁₄)-aryl radical, (C₆-C₁₄) -aryl- (C₁-C₄) -alkyl radical or a (C₂-C₁₀)-heteroaryl or (C₂-C₁₀)-heteroaryl-(C₁-C₄)-alkyl radical which contains one or more heteroatoms selected from the group consisting of N, O and S, where the (C₁-C₄)-alkyl radical may be unsubstituted or mono-polysubstituted by identical or different substituents from the group consisting of (C₁-C₆)-alkyl, halogen and oxo (=O) and where the (C₆-C₁₄)-aryl or (C₂-C₁₀)-heteroaryl radical may be unsubstituted or mono- or polysubstituted by identical or different substituents from the group consisting of straight-chain or branched (C₁-C₈)-alkyl, (C₃-C₇)-cycloalkyl, halogen, cyano, (C1-C6)-alkoxycarbonylamino, (C1-C6)-alkoxy, carboxyl, (C₁-C₈)-alkoxycarbonyl, straight-chain or branched (C₁-C₆)-alkyl which is substituted by one or more fluorine atoms, hydroxyl, straight-chain or branched (C₁-C₈)-alkoxy, where adjacent oxygen atoms may also be linked by (C₁-C₂)-alkylene groups, benzyloxy, nitro, amino, mono-(C₁-C₄)-alkylamino, di-(C₁-C₄)-alkylamino, aryl, which for its part may be unsubstituted or mono- or polysubstituted by identical or different substituents from the group consisting of straight-chain or branched (C₁-C₈)-alkyl, (C₃-C₇)-cycloalkyl, carboxyl, straight-chain or branched (C₁-C₈)-alkoxycarbonyl, by trifluoromethyl, hydroxyl, straight-chain or branched (C₁-C₈)-alkoxy, benzyloxy, nitro, amino, mono-(C₁-C₄)-alkylamino, di-(C₁-C₄)-alkylamino, cyano, straight-chain or branched cyano-(C₁-C₆)-alkyl;
and their structural isomers and stereoisomers, in particular tautomers, diastereomers and enantiomers, and their pharmaceutically acceptable salts, in particular acid addition salts.

2. Acridine derivatives of the formula 1 according to Claim 1, **characterized in that** R, R1, R2, R3, X, Z, P, Q, n and m have the meanings given in Claim 1 and
R4 denotes a straight-chain or branched (C₁-C₂₀)-alkyl radical which may be saturated or unsaturated, with one to three double and/or triple bonds, and which may be unsubstituted or optionally substituted on the same or different C atoms by one, two or more aryl, heteroaryl, halogen, (C1-C6)-alkoxy, amino, mono-(C₁-C₄)-alkylamino or di-(C₁-C₄)-alkylamino;
a phenyl ring or a naphthyl ring, each of which may be unsubstituted or mono- or poly-substituted by identical or different substituents from the group consisting of straight-chain or branched (C₁-C₈)-alkyl, (C₃-C₇)-cycloalkyl, halogen, cyano, (C₁-C₆)-alkoxycarbonylamino, (C₁-C₆)-alkoxy, carboxyl, (C₁-C₈)-alkoxycarbonyl, straight-chain or branched (C₁-C₆)-alkyl which is substituted by one or more fluorine atoms, hydroxyl, straight-chain or branched (C₁-C₈)-alkoxy, benzyloxy, nitro, amino, mono-(C₁-C₄)-alkylamino, di-(C₁-C₄)-alkylamino, aryl, which for its part may be unsubstituted or mono- or polysubstituted by identical or different substituents from the group consisting of straight-chain or branched (C₁-C₈)-alkyl, (C₃-C₇)-cycloalkyl, carboxyl, straight-chain or branched (C₁-C₈)-alkoxycarbonyl, by trifluoromethyl, hydroxyl, straight-chain or branched (C₁-C₈)-alkoxy, benzyloxy, nitro, amino, mono-(C₁-C₄)-alkylamino, di-(C₁-C₄)-alkylamino, cyano, straight-chain or branched cyano-(C₁-C₆)-alkyl;
a 2-, 4-, 5- or 6-pyrimidinyl radical or 2-, 4-, 5- or 6-pyrimidinyl-(C₁-C₄)-alkyl radical, where the (C₁-C₄)-alkyl radical may be unsubstituted or mono- polysubstituted by identidal or different substituents from the group consisting of (C₁-C₆)-alkyl, halogen and oxo (=O) and the 2-, 4-, 5- or 6-pyrimidinyl radical may be unsubstituted or mono- to trisubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆) -alkyl which is mono- or polysubstituted by fluorine, (C₆-C₁₀)-aryl and (C₆-C₁₀) -aryl- (C₁-C₆) -alkyl;
a 3-, 4-, 5- or 6-pyridazinyl radical or 3-, 4-, 5- or 6-pyridazinyl-(C₁-C₄)-alkyl radical, where the (C₁-C₄)-alkyl radical may be unsubstituted or mono- polysubstituted by identical or different substituents from the group consisting of (C₁-C₆)-alkyl, halogen and oxo (=O) and the 3-, 4-, 5- or 6-pyridazinyl radical may be unsubstituted or mono- to trisubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀) -aryl- (C₁-C₆) -alkyl;
a 2-, 3-, 5- or 6-pyrazinyl radical or 2-, 3-, 5- or 6-pyrazinyl-(C₁-C₄)-alkyl radical, where the (C₁-C₄)-alkyl radical may be unsubstituted or mono- polysubstituted by identical or different substituents from the group consisting of (C₁-C₆)-alkyl, halogen and oxo (=O) and the 2-, 3-, 5- or 6-pyrazinyl radical may be unsubstituted or mono- to trisubstituted by identical or different substituents from the group consisting of hydrogen, (C₁-C₆)-alkyl, halogen, nitro, amino, mono-(C₁-C₆)-alkylamino, di- (C₁-C₆) -alkylamino, hydroxyl, (C₁-C₆) -alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 3-, 4-, 5-, 6-, 7-, or 8-cinnolinyl radical or 3-, 4-, 5-, 6-, 7-, or 8-cinnolinyl-(C₁-C₄)-alkyl radical, where the (C₁-C₄)-alkyl radical may be unsubstituted or mono- polysubstituted by identical or different substituents from the group consisting of (C₁-C₆)-alkyl, halogen and oxo (=O) and the 3-, 4-, 5-, 6-, 7-, or 8-cinnolinyl radical may be unsubstituted or mono- to pentasubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀) -aryl- (C₁-C₆) -alkyl;
a 2-, 4-, 5-, 6-, 7-, or 8-quinazolinyl radical or 2-, 4-, 5-, 6-, 7-, or 8-quinazolinyl-(C₁-C₄)-alkyl radical, where the (C₁-C₄)-alkyl radical may be unsubstituted or monopolysubstituted by identidal or different substituents from the group consisting of hydrogen, (C₁-C₆)-alkyl, halogen and oxo (=O) and the or 2-, 4-, 5-, 6-, 7-, or 8-quinazolinyl radical may be unsubstituted or mono- to pentasubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 2-, 3-, 5-, 6-, 7-, or 8-quinoxalinyl radical 2-, 3-, 5-, 6-, 7-, or 8-quinoxalinyl-(C₁-C₄)-alkyl radical, where the (C₁-C₄)-alkyl radical may be unsubstituted or mono- polysubstituted by identidal or different substituents from the group consisting of (C₁-C₆)-alkyl, halogen and oxo (=O) and the or 2-, 3-, 5-, 6-, 7-, or 8-quinoxalinyl radical may be unsubstituted or mono- to pentasubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆) -alkyl;
a 1-, 4-, 5-, 6-, 7-, or 8-phthalazinyl radical or 1-, 4-, 5-, 6-, 7-, or 8-phthalazinyl-(C₁-C₄)-alkyl radical, where the (C₁-C₄)-alkyl radical may be unsubstituted or monopolysubstituted by identidal or different substituents from the group consisting of (C₁-C₆)-alkyl, halogen and oxo (=O) and the or 1-, 4-, 5-, 6-, 7-, or 8-phthalazinyl radical may be unsubstituted or mono- to pentasubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 2-, 3-, 4-, 5-, 6-, 7- or 8-quinolyl radical or 2-, 3-, 4-, 5-, 6-, 7 or 8-quinolyl-(C₁-C₄)-alkyl radical, where the (C₁-C₄)-alkyl radical may be unsubstituted or mono- or poly-substituted by identidal or different substituents from the group consisting of (C₁-C₆)-alkyl, halogen and oxo (=O) and the 2-, 3-, 4-, 5-, 6-, 7- or 8-quinolyl radical may be unsubstituted or mono- to hexasubstituted by identical or different substituents from the group consisting of hydrogen, (C₁-C₆)-alkyl, halogen, nitro, amino, mono- (C₁-C₆) -alkylamino, di- (C₁-C₆) -alkylamino, hydroxyl, (C₁-C₆) -alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 1-, 3-, 4-, 5-, 6-, 7- or 8-isoquinolyl radical or 1-, 3-, 4-, 5-, 6-, 7- or 8-isoquinolyl-(C₁-C₄)-alkyl radical, where the (C₁-C₄)-alkyl radical may be unsubstituted or mono- or polysubstituted by identidal or different substituents from the group consisting of (C₁-C₆)-alkyl, halogen and oxo (=O) and the 1-, 3-, 4-, 5-, 6-, 7- or 8-isoquinolyl radical may be unsubstituted or mono- to hexasubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 2-, 6-, 8- or 9-[9*H*]-purinyl radical or 2-, 6-, 8- or 9-[9*H*]-purinyl-(C₁-C₄)-alkyl radical, where the (C₁-C₄)-alkyl radical may be unsubstituted or mono- or polysubstituted by identidal or different substituents from the group consisting of (C₁-C₆)-alkyl, halogen and oxo (=O) and the 2-, 6-, 8- or 9-[9*H*]-purinyl radical may be unsubstituted or mono- to trisubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonyl-amino or (C₁-C₆)-alkyl which is mono- or polysubstituted by fluorine, (C₆-C₁₀)-aryl and (C₆-C₁₀) -aryl- (C₁-C₆) -alkyl;
a 2-, 6-, 7- or 8-[7*H*]-purinyl radical or 2-, 6-, 7- or 8- [7*H*] -purinyl- (C₁-C₄) -alkyl radical, where the (C₁-C₄)-alkyl radical may be unsubstituted or mono- or polysubstituted by identidal or different substituents from the group consisting of (C₁-C₆)-alkyl, halogen and oxo (=O) and the 2-, 6-, 7- or 8-[7*H*]-purinyl radical may be unsubstituted or mono- to trisubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 1-, 2-, 3-, 4-, 5-, 6-, 7-, 8- or 9-acridinyl radical or 1-, 2-, 3-, 4-, 5-, 6-, 7-, 8- or 9-acridinyl-(C₁-C₄)-alkyl radical, where the (C1-C6)-alkyl radical may be unsubstituted or mono- or polysubstituted by identidal or different substituents from the group consisting of (C₁-C₆)-alkyl, halogen and oxo (=O) and the 1-, 2-, 3-, 4-, 5-, 6-, 7-, 8- or 9-acridinyl radical may be unsubstituted or mono- to octasubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 1-, 2-, 3-, 4-, 5-, 6-, 7-, 8- or 9-phenanthridinyl radical or 1-, 2-, 3-, 4-, 5-, 6-, 7-, 8- or 9-phenanthridinyl-(C₁-C₆)-alkyl radical, where the (C₁-C₆)-alkyl radical may be unsubstituted or mono- or polysubstituted by identidal or different substituents from the group consisting of hydrogen, (C₁-C₆)-alkyl, halogen and oxo (=O) and the 1-, 2-, 3-, 4-, 5-, 6-, 7-, 8- or 9-phenanthridinyl radical may be unsubstituted or mono- to octasubstituted by identical or different substituents from the group consisting of (C₁-C₆)-alkyl, halogen, nitro, amino, mono-(C₁-C₆)-alkylamino, di-(C₁-C₆)-alkylamino, hydroxyl, (C₁-C₆)-alkoxy, (C₆-C₁₀) -aryl- (C₁-C₆) -alkoxy, carboxyl, (C₁-C₆-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 2-, 3-, 4-, 5- or 6-pyridyl radical where the 2-, 3-, 4-, 5- or 6-pyridinyl radical may be unsubstituted or mono- to tetrasubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 2-, 3-, 4-, 5- or 6-pyridyl-(C₁-C₆)-alkyl radical, where the (C₁-C₆)-alkyl radical may be unsubstituted or mono- or polysubstituted by identical or different substituents from the group consisting of (C₁-C₆)-alkyl, halogen and oxo (=O) and the 2-, 3-, 4-, 5- or 6-pyridinyl radical may be unsubstituted or mono- to tetrasubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonyl-amino or (C₁-C₆) -alkyl which is mono- or polysubstituted by fluorine, (C₆-C₁₀)-aryl and (C₆-C₁₀)-aryl-(C₁-C₆)-alkyl;
a 2-, 3-, 4- or 5-thienyl radical or 2-, 3-, 4- or 5-thienyl-(C₁-C₆)-alkyl radical, where the (C₁-C₆)-alkyl radical may be unsubstituted or mono- or polysubstituted by identical or different substituents from the group consisting of (C₁-C₆)-alkyl, halogen and oxo (=O) and the 2-, 3-, 4- or 5-thienyl radical may be unsubstituted or mono- to trisubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 2-, 4-, or 5-thiazolyl radical or 2-, 4-, or 5-thiazolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 2-, 4-, or 5-thiazolyl radical may be unsubstituted or mono- or disubstituted by identical or different substituents from the group consisting of hydrogen, (C₁-C₆)-alkyl, halogen, nitro, amino, mono-(C₁-C₆)-alkylamino, di- (C₁-C₆) -alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀) -aryl and (C₆-C₁₀) -aryl- (C₁-C₆) -alkyl;
a 3-, 4-, or 5-isothiazolyl radical or 3-, 4-, or 5-isothiazolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 3-, 4-, or 5-isothiazolyl radical may be unsubstituted or mono- or disubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 2-, 4-, 5-, 6-, or 7-benzothiazolyl radical or 2-, 4-, 5-, 6-, or 7-benzothiazolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 2-, 4-, 5-, 6-, or 7-benzothiazolyl radical may be unsubstituted or mono- to tetrasubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 1-, 2-, 4-, or 5-imidazolyl radical or 1-, 2-, 4-, or 5-imidazolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 1-, 2-, 4-, or 5-imidazolyl radical may be unsubstituted or mono- to trisubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀) -aryl- (C₁-C₆)-alkyl;
a 1-, 3-, 4-, or 5-pyrazolyl radical or 1-, 3-, 4- or 5-pyrazolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 1-, 3-, 4- or 5-pyrazolyl radical may be unsubstituted or mono- to trisubstituted by identical or different substituents from the group consisting of hydrogen, (C₁-C₆)-alkyl, halogen, nitro, amino, mono-(C₁-C₆)-alkylamino, di- (C₁-C₆) -alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 1-, 2-, 3-, 4-, or 5-pyrrolyl radical or 1-, 2-, 3-, 4-, or 5-pyrrolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 1-, 2-, 3-, 4- or 5-pyrrolyl radical may be unsubstituted or mono- to tetrasubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀) -aryl- (C₁-C₆) -alkyl;
a 1-, 3-, or 5-[1.2.4]-triazolyl radical or 1-, 3-, or 5-[1.2.4]-triazolyl-(C₁-C₆)-alkyl radical, where the (C₁-C₆)-alkyl radical may be unsubstituted or mono- or polysubstituted by identidal or different substituents from the group consisting of hydrogen, (C₁-C₆)-alkyl, halogen and oxo (=O) and the 1-, 3-, or 5-[1.2.4]-triazolyl radical may be unsubstituted or mono- or disubstituted by identical or different substituents from the group consisting of (C₁-C₆)-alkyl, halogen, nitro, amino, mono- (C₁-C₆)-alkylamino, di- (C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 1-, 4-, or 5-[1.2.3]-triazolyl radical or 1-, 4-, or 5-[1.2.3]-triazolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 1-, 4-, or 5-[1.2.3]-triazolyl radical may be unsubstituted or mono- or disubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆) -alkoxycarbonyl, (C₁-C₆) -alkoxycarbonyl-amino or (C₁-C₆)-alkyl which is mono- or polysubstituted by fluorine, (C₆-C₁₀)-aryl and (C₆-C₁₀) -aryl- (C₁-C₆) -alkyl;
a 1- or 5-[1*H*]-tetrazolyl radical or 1- or 5-[1*H*]-tetrazolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 1- or 5-[1*H*]-tetrazolyl radical may be unsubstituted or substituted by hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆) -alkyl which is mono- or polysubstituted by fluorine, (C₆-C₁₀)-aryl and (C₆-C₁₀) -aryl- (C₁-C₆)-alkyl;
a 2- or 5-[2*H*]-tetrazoyl radical or 2- or 5-[2*H*]-tetrazolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 2- or 5-[2*H*]-tetrazolyl radical may be unsubstituted or substituted by hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀) -aryl and (C₆-C₁₀) -aryl- (C₁-C₆) -alkyl;
a 2-, 4-, or 6-[1.3.5]-triazinyl radical or 2-, 4-, or 6-[1.3.5]-triazinyl-(C₁-C₆)-alkyl radical, where the (C₁-C₆)-alkyl radical may be unsubstituted or mono- or polysubstituted by identidal or different substituents from the group consisting of hydrogen,(C₁-C₆)-alkyl, halogen and oxo (=O) and the 2-, 4-, or 6-[1.3.5]-triazinyl radical may be unsubstituted or mono- or disubstituted by identical or different substituents from the group consisting of hydrogen, (C₁-C₆)-alkyl, halogen, nitro, amino, mono-(C₁-C₆)-alkylamino, di-(C₁-C₆) -alkylamino, hydroxyl, (C₁-C₆) -alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl;
a 2-, 4-, or 5-oxazolyl radical or 2-, 4-, or 5-oxazolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 2-, 4-, or 5-oxazolyl radical may be unsubstituted or mono- or disubstituted by identical or different substituents from the group consisting of hydrogen, (C₁-C₆)-alkyl, halogen, nitro, amino, mono-(C₁-C₆)-alkylamino, di-(C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀) -aryl- (C₁-C₆) -alkyl;
a 3-, 4-, or 5-isoxazolyl radical or 3-, 4-, or 5-isoxazolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 3-, 4-, or 5-isoxazolyl radical may be unsubstituted or mono- or disubstituted by identical or different substituents from the group consisting of hydrogen, (C₁-C₆)-alkyl, halogen, nitro, amino, mono-(C₁-C₆)-alkylamino, di- (C₁-C₆) -alkylamino, hydroxyl, (C₁-C₆)-alkoxy, benzyloxy, carboxyl, (C₁-C₆)-alkoxycarbonyl, (C₁-C₆) -alkoxycarbonylamino or (C₁-C₆) -alkyl
which is mono- or polysubstituted by fluorine, (C₆-C₁₀)-aryl and (C₆-C₁₀)-aryl-(C₁-C₆)-alkyl;
a 1-, 2-, 3-, 4-, 5-, 6- or 7-indolyl radical or 1-, 2-, 3-, 4-, 5-, 6- or 7-indolyl-(C₁-C₆)-alkyl radical, where the (C₁-C₆)-alkyl radical may be unsubstituted or mono- or polysubstituted by identidal or different substituents from the group consisting of (C₁-C₆)-alkyl, halogen and oxo (=O) and the 1-, 2-, 3-, 4-, 5-, 6- or 7-indolyl radical may be unsubstituted or mono- to hexasubstituted by identical or different substituents from the group consisting of hydrogen, (C₁-C₆)-alkyl, halogen, nitro, amino, mono-(C₁-C₆)-alkylamino, di- (C₁-C₆)-alkylamino, hydroxyl, (C₁-C₆)-alkoxy, benzyloxy, carboxyl, (C₁-C₆)-alkoxycarbonyl, (C₁-C₆)-alkoxycarbonylamino or (C₁-C₆)-alkyl which is mono- or polysubstituted by fluorine, (C₆-C₁₀)-aryl and (C₆-C₁₀)-aryl-(C₁-C₆)-alkyl.

3. Acridine derivatives according to Claim 1 or 2, **characterized in that** R, R₁, R₂, R₃, X, Z, P, Q, n and m have the meanings given above and R4 represents phenyl which is unsubstituted or substituted by one to five identical or different (C₁-C₆)-alkoxy groups, where adjacent oxygen atoms may also be linked by (C₁-C₂)-alkylene groups.

4. Acridine derivatives according to any of Claims 1 to 3, **characterized in that** R, R₁, R₂, R₃, P, Q, X, Z, n and m have the meanings given above and R₄ represents 3,5-dimethoxyphenyl.

5. Acridine derivatives according to any of the preceding claims, **characterized in that** R₄ has the meanings given above, R, R₁, R₂, R₃ each represent a hydrogen atom, Z represents an oxygen atom and X represents a nitrogen atom, P and Q each represent two hydrogen atoms and m is zero and n represents the integer 2.

6. Acridine derivatives according to any of the preceding claims, **characterized in that** R, R₁, R₂, R₃ each represent a hydrogen atom, Z represents an oxygen atom and X represents a nitrogen atom, P and Q each represent two hydrogen atoms and m is zero and n represents the integer 2, and R₄ represents 3,5-dimethoxyphenyl.

7. Acridine derivatives according to any of Claims 1 to 6 for use as medicaments.

8. Use of the acridine derivatives according to any of Claims 1 to 6 for preparing a medicament for treating tumors in mammals.

9. Process for preparing acridine derivatives according to any of Claims 1 to 6, **characterized in that** an acridine carboxylic acid of the formula (2) in which R, R1, R2, R3 have the meanings given above, Z denotes an oxygen or sulfur atom and Y represents a leaving group such as halogen, hydroxyl, (C1-C6)-alkoxy, -O-tosyl, -O-mesyl or imidazolyl, 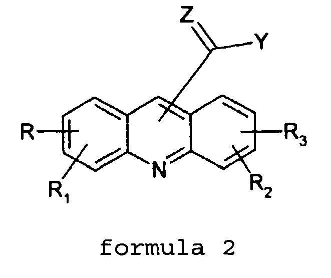 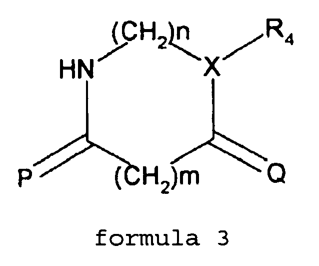 is reacted with an amine of the formula (3) in which R4, X, P, Q, m and n have the meanings given above, if appropriate using diluents and auxiliaries, and the desired acridine derivative is formed.

10. Medicament, **characterized in that** it comprises, as active ingredient, at least one acridine derivative according to any of Claims 1 to 6, if appropriate together with customary pharmaceutically acceptable auxiliaries, additives and carriers.

## Revendications

1. dérivés d'acridine selon la formule générale 1 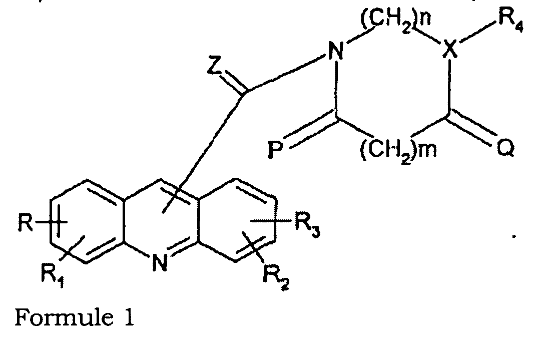 dans laquelle
R, R₁, R₂, R₃ peuvent être liés, au choix, aux atomes de carbone C₁ à C₉ de l'acridine, sont identiques ou différents et représentent, indépendamment l'un de l'autre, un groupe (C₁ à C₈)-alkyle linéaire ou ramifié, (C₃ à C₇)-cycloalkyle, (C₁ à C₈)-alkylcarbonyle linéaire ou ramifié, un groupe (C₁ à C₈)-alcoxy linéaire ou ramifié, halogène, aryle-(C₁ à C₈)-alcoxy, nitro, amino, mono-(C₁ à C₄)-alkylamino, di-(C₁ à C₄)-alkylamino, (C₁ à C₈)-alcoxycarbonyle-amino, (C₁ à C₆)-alcoxycarbonylamino-(C₁ à C₈)-alkyle, cyano, cyano-(C₁ à C₆)-alkyle à chaîne droite ou ramifié, carboxy, (C₁ à C₈)-alcoxycarbonyle, un groupe (C₁ à C₄)-alkyle substitué par un ou plusieurs atomes de fluor, un groupe carboxy-(C₁ à C₈)-alkyle ou (C₁ à C₈)-alcoxycarbonyle-(C₁ à C₆)-alkyle, (C₂ à C₆)-alcényle, (C₂ à C₆)-alkynyle, un groupe cyano-(C₁ à C₆)-alkyle à chaîne droite ou ramifié, le radical aryle pouvant être non substitué ou substitué une ou plusieurs fois de manière identique ou différente par un groupe halogène, (C₁ à C₈)-alkyle à chaîne droite ou ramifié, (C₃ à C₇)-cycloalkyle, carboxy, (C₁ à C₈)-alcoxycarbonyle à chaîne droite ou ramifié, par un groupe trifluorométhyle, hydroxy, (C₁ à C₈)-alcoxy à chaîne droite ou ramifié, benzyloxy, nitro, amino mono-(C₁ à C₄)-alkylamino, di-(C₁ à C₄)-alkylamino, cyano, cyano-(C₁ à C₆)-alkyle à chaîne droite ou ramifié,
Z est un atome d'oxygène ou de soufre, le radical 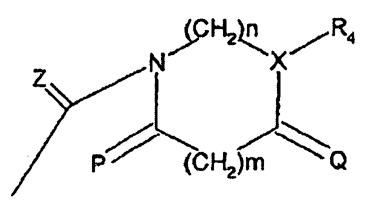 substitué sur l'hétérocycle d'acridine pouvant être lié aux atomes de C, C₁ à C₉ du squelette cyclique de l'acridine ;
P, Q représentent, indépendamment l'un de l'autre, un atome d'oxygène ou, respectivement, deux atomes d'hydrogène ;
X représente un atome d'azote ou C-R₅, R₅ représentant un atome d'hydrogène ou un groupe (C₁ ou C₆)-alkyle ;
n, m représentent, indépendamment l'un de l'autre, un nombre entier compris entre 0 et 3, à condition que, dans le cas où n=0, X représente un groupe CR₅R₆, R₅ et R₆ représentant, indépendamment l'un de l'autre, un atome d'hydrogène ou un groupe (C₁ à C₆)-alkyle et un atome d'hydrogène ou un groupe (C₁ à C₆)-alkyle est substitué sur l'atome d'azote voisin du groupe C=Z,
R₄ représente un radical (C₁ à C₂₀)-alkyle à chaîne droite ou ramifié, lequel peut être saturé ou bien insaturé avec une à trois liaisons doubles et/ou triples et qui peut être non substitué ou bien substitué au choix sur le même atome de C ou bien des atomes de C différents, par un, deux ou plusieurs groupes aryle, hétéroaryle, halogène, cyano, (C₁ à C₆)-alcoxycarbonylamino, (C1 à C6)-alcoxy, amino, mono-(C₁ à C₄)-alkylamino ou di-(C₁ à C₄)-alkylamino ; un radical (C₆ à C₁₄)-aryle, un radical (C₆ à C₁₄)-aryle-(C₁ à C₄)-alkyle ou un radical (C₂ à C₁₀)-hétéroaryle ou (C₂ à C₁₀)-hétéroaryle-(C₁ à C₄)-alkyle contenant un ou plusieurs hétéroatomes choisis dans le groupe N, O et S, le radical (C₁ à C₄)-alkyle pouvant être non substitué ou substitué une ou plusieurs fois, de manière identique ou différente, par un groupe (C₁ à C₆)-alkyle, halogène ou oxo (= O) et le radical (C₆ à C₁₄)-aryle ou (C₂ à C₁₀)-hétéroaryle pouvant être non substitué ou bien substitué une ou plusieurs fois, de manière identique ou différente, par un groupe (C₁ à C₈)-alkyle à chaîne droite ou ramifié, (C₃ à C₇)-cycloalkyle, halogène, cyano, (C₁ à C₆)-alcoxycarbonylamino, (C₁ à C₆)-alcoxy, carboxy, (C₁ à C₈)-alcoxycarbonyle, un groupe (C₁ à C₆)-alkyle à chaîne droite ou ramifié, substitué par un ou plusieurs atomes de fluor, hydroxy, (C₁ à C₈)-alcoxy à chaîne droite ou ramifié, des atomes d'oxygène voisins pouvant être également associés par des groupes (C₁ à C₂)-alkylène, un groupe benzyloxy, nitro, amino, mono- (C₁ à C₄)-alkylamino, di-(C₁ à C₄)-alkylamino, aryle, lequel peut être, de son côté, non substitué ou substitué une ou plusieurs fois, de manière identique ou différente, par un groupe (C₁ à C₈)-alkyle à chaîne droite ou ramifié, (C₃ à C₇)-cycloalkyle, carboxy, un groupe (C₁ à C₈)-alcoxycarbonyle à chaîne droite ou ramifié, par un groupe trifluorométhyle, hydroxy, (C₁ à C₈)-alcoxy à chaîne droite ou ramifié, benzyloxy, nitro, amino, mono-(C₁ à C₄)-alkylamino, di-(C₁ à C₄)-alkylamino, cyano, cyano-(C₁ à C₆)-alkyle à chaîne droite ou ramifié ;
ainsi que leurs isomères structurels et stéréo-isomères, en particulier les tautomères, les diastéréomères et les énantiomères, et leurs sels compatibles au plan pharmaceutique.

2. Dérivés d'acridine de formule générale 1 selon la revendication 1,
**caractérisés en ce que**
R, R1, R2, R3, X, Z, P, Q, n et m ont les significations indiquées dans la revendication 1 et
R₄ représente un radical (C₁ à C₂₀)-alkyle à chaîne droite ou ramifié, qui peut être saturé ou insaturé avec une à trois liaisons doubles et/ ou triples et qui peut être non substitué ou, au choix, substitué sur le même atome de C ou des atomes de C différents par un, deux ou plusieurs groupes aryle, hétéroaryle, halogène, (C1 à C6)-alcoxy, amino, mono-(C₁ à C₄)- alkylamino ou di-(C₁ à C₄)-alkylamino ;
un cycle phényle ou naphtyle qui peuvent être respectivement non substitués ou substitués une ou plusieurs fois de manière identique ou différente, par un groupe (C₁ à C₈)-alkyle à chaîne droite ou ramifié, (C₃ à C₇)-cycloalkyle, halogène, cyano, (C₁ à C₆)-alcoxycarbonylamino, (C₁ à C₆)-alcoxy, carboxy, (C₁ à C₈)-alcoxycarbonyle, par un groupe (C₁ à C₆)-alkyle à chaîne droite ou ramifié, substitué par un ou plusieurs atomes de fluor, hydroxy, (C₁ à C₈)-alcoxy à chaîne droite ou ramifié, benzyloxy, nitro, amino, mono-(C₁ à C₄)-alkylamino, di-(C₁ à C₄)-alkylamino, aryle, lequel peut être, de son côté, non substitué ou substitué une ou plusieurs fois, de manière identique ou différente, par un groupe (C₁ à C₈)-alkyle à chaîne droite ou ramifié, (C₃ à C₇)-cycloalkyle, carboxy, un groupe (C₁ à C₈)-alcoxycarbonyle à chaîne droite ou ramifié, par un groupe trifluorométhyle, hydroxy, (C₁ à C₈)-alcoxy à chaîne droite ou ramifié, benzyloxy, nitro, amino, mono-(C₁ à C₄)-alkylamino, di-(C₁ à C₄)-alkylamino, cyano, cyano-(C₁ à C₆)-alkyle à chaîne droite ou ramifié,
un radical 2-, 4-, 5- ou 6-pyrimidinyle ou un radical 2-, 4-, 5- ou 6-pyrimidinyle-(C₁ à C₄)-alkyle, le radical (C₁ à C₄)-alkyle pouvant être non substitué ou substitué une ou plusieurs fois, de manière identique ou différente, par un groupe (C₁ à C₆)-alkyle, halogène ou oxo (=O), et le radical 2-, 4-, 5- ou 6-pyrimidinyle pouvant être non substitué ou substitué une à trois fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 3-, 4-, 5- ou 6-pyridazinyle ou un radical 3-, 4-, 5- ou 6-pyridazinyle-(C₁ à C₄)-alkyle, le radical (C₁ à C₄)-alkyle pouvant être non substitué ou substitué une ou plusieurs fois, de manière identique ou différente, par un groupe (C₁ à C₆)-alkyle, halogène ou oxo (=O), et le radical 3-, 4-, 5- ou 6-pyridazinyle pouvant être non substitué ou substitué une à trois fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, de préférence un groupe trifluorométhyle, (C₆ à C₁₀)-aryle ou (C₆ à C₁₀)-aryle-(C₁ à C₆)-alkyle ;
un radical 2-, 3-, 5- ou 6-pyrazinyle ou un radical 2-, 3-, 5- ou 6-pyrazinyle-(C₁ à C₄)-alkyle, le radical (C₁ à C₄)-alkyle pouvant être non substitué ou substitué une ou plusieurs fois, de manière identique ou différente, par un groupe (C₁ à C₆)-alkyle, halogène ou oxo (=O), et le radical 2-, 3-, 5- ou 6-pyrazinyle pouvant être non substitué ou substitué une à trois fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 3-, 4-, 5-, 6-, 7- ou 8-cinnolinyle ou un radical 3-, 4-, 5-, 6-, 7- ou 8-cinnolinyle-(C₁ à C₄)-alkyle, le radical (C₁ à C₄)-alkyle pouvant être non substitué ou substitué une ou plusieurs fois de manière identique ou différente par un groupe (C₁ à C₆)-alkyle, halogène ou oxo (=O), et le radical 3-, 4-, 5-, 6-, 7- ou 8-cinnolinyle pouvant être non substitué ou substitué une à cinq fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 4-, 5-, 6-, 7- ou 8-quinazolinyle ou un radical 2-, 4-, 5-, 6-, 7- ou 8-quinazolinyle-(C₁ à C₄)-alkyle, le radical (C₁ à C₄)-alkyle pouvant être non substitué ou substitué une ou plusieurs fois de manière identique ou différente par un atome d'hydrogène, un groupe (C₁ à C₆)-alkyle, halogène ou oxo (=O), et le radical 2-, 4-, 5-, 6-, 7- ou 8-quinazolinyle pouvant être non substitué ou substitué une à cinq fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 3-, 5-, 6-, 7- ou 8-quinoxalinyle ou un radical 2-, 3-, 5-, 6-, 7- ou 8-quinoxalinyle-(C₁ à C₄)-alkyle, le radical (C₁ à C₄)-alkyle pouvant être non substitué ou substitué une ou plusieurs fois de manière identique ou différente par un atome d'hydrogène, un groupe (C₁ à C₆)-alkyle, halogène ou oxo (=O), et le radical 2-, 3-, 5-, 6-, 7- ou 8-quinoxalinyle pouvant être non substitué ou substitué une à cinq fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 1-, 4-, 5-, 6-, 7- ou 8-phtalazinyle ou un radical 1-, 4-, 5-, 6-, 7- ou 8-phtalazinyle-(C₁ à C₄)-alkyle, le radical (C₁ à C₄)-alkyle pouvant être non substitué ou substitué une ou plusieurs fois de manière identique ou différente par un groupe (C₁ à C₆)-alkyle, halogène ou oxo (=O), et le radical 1-, 4-, 5-, 6-, 7- ou 8-phtalazinyle pouvant être non substitué ou substitué une à cinq fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 3-, 4-, 5-, 6-, 7- ou 8-quinolyle ou un radical 2-, 3-, 4-, 5-, 6-, 7- ou 8-quinolyle-(C₁ à C₄)-alkyle, le radical (C₁ à C₄)-alkyle pouvant être non substitué ou substitué une ou plusieurs fois de manière identique ou différente par un groupe (C₁ à C₆)-alkyle, halogène ou oxo (=O), et le radical 2-, 3-, 4-, 5-, 6-, 7- ou 8-quinolyle pouvant être non substitué ou substitué une à six fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 1-, 3-, 4-, 5-, 6-, 7- ou 8-isoquinolyle ou un radical 1-, 3-, 4-, 5-, 6-, 7- ou 8-isoquinolyle-(C₁ à C₄)-alkyle, le radical (C₁ à C₄)-alkyle pouvant être non substitué ou substitué une ou plusieurs fois de manière identique ou différente par un groupe (C₁ à C₆)-alkyle, halogène ou oxo (=O), et le radical 1-, 3-, 4-, 5-, 6-, 7- ou 8-isoquinolyle pouvant être non substitué ou substitué une à six fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 6-, 8- ou 9-[*9H*]-purinyle ou un radical 2-, 6-, 8- ou 9-[*9H*]-purinyle-(C₁ à C₄)-alkyle, le radical (C₁ à C₄)-alkyle pouvant être non substitué ou substitué une ou plusieurs fois de manière identique ou différente par un groupe (C₁ à C₆)-alkyle, halogène ou oxo (=O), et le radical 2-, 6-, 8- ou 9-[9*H*]-purinyle pouvant être non substitué ou substitué une à trois fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 6-, 7- ou 8-[*7H*]-purinyle ou un radical 2-, 6-, 7- ou 8-[*7H*]-purinyle-(C₁ à C₄)-alkyle, le radical (C₁ à C₄)-alkyle pouvant être non substitué ou substitué une ou plusieurs fois de manière identique ou différente par un groupe (C₁ à C₆)-alkyle, halogène ou oxo (=O), et le radical 2-, 6-, 7-ou 8-[*7H*]-purinyle pouvant être non substitué ou substitué une à trois fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 1-, 2-, 3-, 4-, 5-, 6-, 7-, 8- ou 9-acridinyle ou un radical 1-, 2-, 3-, 4-, 5-, 6-, 7-, 8- ou 9-acridinyle-(C₁ à C₄)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 1-, 2-, 3-, 4-, 5-, 6-, 7-, 8- ou 9-acridinyle pouvant être non substitué ou substitué une à huit fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 1-, 2-, 3-, 4-, 5-, 6-, 7-, 8- ou 9-phénanthridinyle ou un radical 1-, 2-, 3-, 4-, 5-, 6-, 7-, 8- ou 9-phénanthridinyle-(C₁ à C₆)-alkyle, le radical (C₁ à C₆)-alkyle pouvant être non substitué ou substitué une ou plusieurs fois de manière identique ou différente par un atome d'hydrogène, un groupe (C₁ à C₆)-alkyle, halogène ou oxo (=O), et le radical 1-, 2-, 3-, 4-, 5-, 6-, 7-, 8- ou 9-phénanthridinyle pouvant être non substitué ou substitué une à huit fois de manière identique ou différente un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, (C₆ à C₁₀)-aryle-(C₁ à C₆)-alcoxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 3-, 4-, 5-, ou 6-pyridyle, le radical 2-, 3-, 4-, 5-, ou 6-pyridinyle pouvant être non substitué ou substitué une à quatre fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 3-, 4-, 5-, ou 6-pyridyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), le radical 2-, 3-, 4-, 5-, ou 6-pyridinyle pouvant être non substitué ou substitué une à quatre fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 3-, 4-, ou 5-thiényle ou un radical 2-, 3-, 4-, ou 5-thiényle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 2-, 3-, 4-, ou 5-thiényle pouvant être non substitué ou substitué une à trois fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 4-, ou 5-thiazolyle ou un radical 2-, 4-, ou 5-thiazolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 2-, 4-, ou 5-thiazolyle pouvant être non substitué ou substitué une ou deux fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 3-, 4-, ou 5-isothiazolyle ou un radical 3-, 4-, ou 5-isothiazolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 3-, 4-, ou 5-isothiazolyle pouvant être non substitué ou substitué une ou deux fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 4-, 5-, 6- ou 7-benzothiazolyle ou un radical 2-, 4-, 5-, 6- ou 7-benzothiazolyle-(C₁ à C₆)-alkyle, le radical (C₁ à C₆)-alkyle pouvant être pouvant être non substitué ou substitué une ou plusieurs fois de manière identique ou différente par un groupe (C₁ à C₆)-alkyle, halogène ou oxo (=O) et le radical 2-, 4-, 5-, 6- ou 7-benzothiazolyle non substitué ou substitué une à quatre fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, de préférence un groupe trifluorométhyle, (C₆ à C₁₀)-aryle ou (C₆ à C₁₀)-aryle-(C₁ à C₆)-alkyle ;
un radical 1-, 2-, 4-, ou 5-imidazolyle ou un radical 1-, 2-, 4-, ou 5-imidazolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 1-, 2-, 4-, ou 5-imidazolyle pouvant être non substitué ou substitué une à trois fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, de préférence un groupe trifluorométhyle, (C₆ à C₁₀)-aryle ou (C₆ à C₁₀)-aryle-(C₁ à C₆)-alkyle ;
un radical 1-, 3-, 4-, ou 5-pyrazolyle ou un radical 1-, 3-, 4-, ou 5-pyrazolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 1-, 3-, 4-, ou 5-pyrazolyle pouvant être non substitué ou substitué une à trois fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 1-, 2-, 3-, 4-, ou 5-pyrrolyle ou un radical 1-, 2-, 3-, 4-, ou 5-pyrrolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 1-, 2-, 3-, 4-, ou 5-pyrrolyle pouvant être non substitué ou substitué une à quatre fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 1-, 3-, ou 5-[1.2.4]-triazolyle ou un radical 1-, 3-, ou 5-[1.2.4]-triazolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 1-, 3-, ou 5-[1.2.4]-triazolyle pouvant être non substitué ou substitué une ou deux fois de manière identique ou différente par un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 1-, 4-, ou 5-[1.2.3]-triazolyle ou un radical 1-, 4-, ou 5-[1.2.3]-triazolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 1-, 4-, ou 5-[1.2.3]-triazolyle pouvant être non substitué ou substitué une ou deux fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 1- ou 5-[1*H*]-tétrazolyle ou un radical 1- ou 5-[1*H*]-tétrazolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 1- ou 5-[1*H*]-tétrazolyle pouvant être non substitué ou substitué par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2- ou 5-[2*H*]-tétrazolyle ou un radical 2- ou 5-[2*H*]-tétrazolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 2- ou 5-[2*H*]-tétrazolyle pouvant être non substitué ou substitué par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 4-, ou 6-[1.3.5]-triazinyle ou un radical 2-, 4-, ou 6-[1.3.5]-triazinyle-(C₁ à C₆)-alkyle, le radical (C₁ à C₆)-alkyle pouvant être non substitué ou substitué une ou plusieurs fois de manière identique ou différente par un atome d'hydrogène, un groupe (C₁ à C₆)-alkyle, halogène ou oxo (=O), et le radical 2-, 4-, ou 6-[1.3.5]-triazinyle pouvant être non substitué ou substitué une ou deux fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 2-, 4-, ou 5-oxazolyle ou un radical 2-, 4-, ou 5-oxazolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 2-, 4-, ou 5-oxazolyle pouvant être non substitué ou substitué une ou deux fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 3-, 4-, ou 5-isoxazolyle ou un radical 3-, 4-, ou 5-isoxazolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 3-, 4-, ou 5-isoxazolyle pouvant être non substitué ou substitué une ou deux fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle ;
un radical 1-, 2-, 3-, 4-, 5-, 6- ou 7-indolyle ou un radical 1-, 2-, 3-, 4-, 5-, 6- ou 7-indolyle-(C₁ à C₆)-alkyle, le radical (C₁ à C₆)-alkyle pouvant être non substitué ou substitué une ou plusieurs fois de manière identique ou différente par un groupe (C₁ à C₆)-alkyle, halogène ou oxo (=O), et le radical 1-, 2-, 3-, 4-, 5-, 6- ou 7-indolyle pouvant être non substitué ou substitué une à six fois de manière identique ou différente par un atome d'hydrogène, un groupe (C₁ à C₆)-alkyle, halogène, nitro, amino, mono-(C₁ à C₆)-alkylamino, di-(C₁ à C₆)-alkylamino, hydroxy, (C₁ à C₆)-alcoxy, benzyloxy, carboxy, (C₁ à C₆)-alcoxycarbonyle, (C₁ à C₆)-alcoxycarbonylamino ou un groupe (C₁ à C₆)-alkyle substitué une ou plusieurs fois par du fluor, (C₆ à C₁₀)-aryle ou (C₆ à C₁₀)-aryle-(C₁ à C₆)-alkyle.

3. Dérivés d'acridine selon la revendication 1 ou 2,
**caractérisés en ce que**
R, R₁, R₂, R₃, X, Z, P, Q, n et m ont les significations indiquées précédemment et R4 représente un groupe phényle, non substitué ou substitué par un à cinq groupes (C₁-C₆)-alcoxy identiques ou différents, des atomes d'oxygène voisins pouvant être également associés par des groupes alkylène (C₁-C₂).

4. Dérivés d'acridine selon l'une quelconque des revendications 1 à 3,
**caractérisés en ce que**
R, R₁, R₂, R₃, P, Q, X, Z, n et m ont les significations indiquées précédemment et R₄ représente un groupe 3,5-diméthoxyphényle.

5. Dérivés d'acridine selon l'une quelconque des revendications précédentes,
**caractérisés en ce que**
R₄ a les significations indiquées précédemment et R, R₁, R₂, R₃ représentent respectivement un atome d'hydrogène, Z un atome d'oxygène et X un atome d'azote, P et Q respectivement deux atomes d'hydrogène, m est égal à zéro et n est le nombre entier 2.

6. Dérivés d'acridine selon l'une quelconque des revendications précédentes,
**caractérisés en ce que**
R, R₁, R₂ et R₃ représentent respectivement un atome d'hydrogène, Z un atome d'oxygène et X un atome d'azote, P et Q respectivement deux atomes d'hydrogène, m est égal à zéro, n est le nombre entier 2 et R₄ représente un radical 3,5-diméthoxyphényle.

7. Dérivés d'acridine selon l'une quelconque des revendications 1 à 6, destinés à être utilisés comme médicament.

8. Utilisation des dérivés d'acridine selon l'une quelconque des revendications 1 à 6, pour la fabrication d'un médicament pour le traitement de tumeurs chez les mammifères.

9. Procédé de fabrication de dérivés d'acridine selon l'une quelconque des revendications 1 à 6,
**caractérisé en ce qu'**
on convertit un acide acridine carboxylique de formule générale (2), dans laquelle R, R1, R2, R3 ont les significations indiquées précédemment, Z représente un atome d'oxygène ou de soufre et Y un groupe partant comme un groupe halogène, hydroxy, (C1-C6) alcoxy, -O-tosyle, -O-mésyle ou imidazolyle, 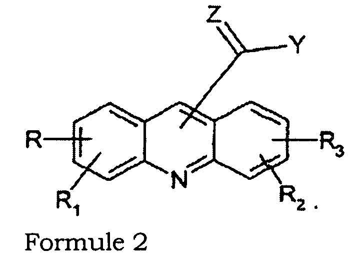 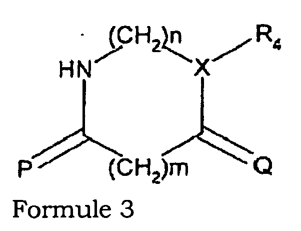 avec une amine de formule générale (3), dans laquelle R4, X, P, Q, m et n ont les significations indiquées précédemment, le cas échéant en utilisant des agents de dilution et des excipients tout en formant les dérivés d'acridine souhaités.

10. Médicament,
**caractérisé en ce que**
comme constituant actif, il contient au moins un dérivé d'acridine selon l'une quelconque des revendications 1 à 6, le cas échéant conjointement avec des excipients, des additifs et des porteurs usuels pharmaceutiquement compatibles.
